# EUROPÄISCHE PATENTANMELDUNG

(11) **EP 3 338 803 A1**
(43) Veröffentlichungstag der Anmeldung: **27.06.2018**
(21) Anmeldenummer: 16205686.5
(22) Anmeldetag: 21.12.2016
(51) Int. Cl.: A61K 47/10, A61K 9/08, A61K 31/496

(54) **PHARMAZEUTISCHE DARREICHUNGSFORMEN ENTHALTEND INHIBITOREN VON TASK-1 UND TASK-3 KANÄLEN UND DEREN VERWENDUNG FÜR DIE THERAPIE VON ATEMSTÖRUNGEN**

(71) Anmelder: Bayer Pharma Aktiengesellschaft, 13353 Berlin (DE)
(72) Erfinder: Die Erfindernennung liegt noch nicht vor
(74) Vertreter: BIP Patents

(57) **Zusammenfassung**

Die vorliegende Anmeldung betrifft neue pharmazeutische Darreichungsformen enthaltend potente und selektive Inhibitoren von TASK-1 und/oder TASK-3 Kanälen und ihre Verwendung für die Behandlung und/oder Prävention von Atemstörungen, einschließlich schlafbedingter Atemstörungen wie obstruktiven und zentralen Schlafapnoen und Schnarchen.

## Beschreibung

Die vorliegende Anmeldung betrifft neue pharmazeutische Darreichungsformen enthaltend potente und selektive Inhibitoren von TASK-1 und/oder TASK-3 Kanälen und ihre Verwendung für die Behandlung und/oder Prävention von Atemstörungen, einschließlich schlafbedingter Atemstörungen wie obstruktiven und zentralen Schlafapnoen und Schnarchen.

Kaliumkanäle sind nahezu ubiquitär vorkommende Membranproteine, die an einer Vielzahl von unterschiedlichen physiologischen Prozessen beteiligt sind. Dazu gehört auch die Regulation des Membranpotentials und der elektrischen Erregbarkeit von Neuronen und Muskelzellen. Kaliumkanäle werden in drei größere Gruppen unterteilt, welche sich in der Zahl der Transmembrandomänen (2, 4 oder 6) unterscheiden. Die Gruppe von Kaliumkanälen, bei der zwei porenbildende Domänen von vier Transmembrandomänen flankiert werden, wird K2P-Kanäle (Two-pore domain *K*⁺) genannt. Funktionell vermitteln die K2P-Kanäle weitgehend zeit- und spannungsunabhängig K⁺-Hintergrundströme und tragen entscheidend zur Aufrechterhaltung des Ruhemembranpotentials bei. Die Familie der K2P-Kanäle umfasst 15 Mitglieder, die in sechs Subfamilien untergliedert sind, basierend auf Ähnlichkeiten in Sequenz, Struktur und Funktion: TWIK (Tandem pore domain halothane inhibited K⁺ channel), TREK (TWIK-related K⁺ channel), TASK (TWIK-related acid-sensitive K⁺ channel), TALK (TWIK-related alkaline pH activated K⁺ channel), THIK (Tandem pore domain halothane inhibited K⁺ channel) und TRESK (TWIK-related spinal cord K⁺ channel).

Von besonderem Interesse sind TASK-1 (KCNK3 oder K2P3.1) und TASK-3 (KCNK9 oder K2P9.1) der TASK (*TWIK-related acid-sensitive K*⁺ *channel*)-Subfamilie. Diese Kanäle sind funktionell dadurch gekennzeichnet, dass durch sie bei Erhaltung der spannungsunabhängigen Kinetik sogenannte "Leck"- oder "Hintergrund"-Ströme fließen, wobei sie auf eine Vielzahl von physiologischen und pathologischen Einflüssen mit einer Zu- oder Abnahme der Aktivität reagieren. Charakteristisch für TASK-Kanäle ist die sensitive Reaktion auf eine Änderung des extrazellulären pH-Wertes: Die Kanäle werden bei saurem pH-Wert inhibiert und bei alkalischem pH-Wert aktiviert.

TASK-1- und TASK-3-Kanäle spielen eine Rolle bei der Regulation der Atmung. Beide Kanäle werden in den respiratorischen Neuronen des Atemzentrums im Hirnstamm exprimiert, unter anderem in Neuronen, die den Atemrhythmus generieren (ventrale respiratorische Gruppe mit dem prä-Bötzinger-Komplex), und im noradrenergen *Locus caeruleus* sowie in serotonergen Neuronen der Raphe-Kerne. Aufgrund der pH-Abhängigkeit übernehmen die TASK-Kanäle hier die Funktion eines Sensors, der extrazelluläre pH-Wert-Änderungen in entsprechende zelluläre Signale übersetzt [Bayliss et al., Pflugers Arch. 467, 917-929 (2015)]. Auch im *Glomus caroticum,* einem Paraganglion, das den pH-Wert und den O₂- und CO₂-Gehalt des Blutes misst und Signale an das Atemzentrum im Hirnstamm übermittelt, um die Atmung zu regulieren, werden TASK-1 und TASK-3 exprimiert. Es wurde gezeigt, dass TASK-1 knock-out-Mäuse eine verringerte ventilatorische Reaktion (Anstieg der Atemfrequenz und des Atemzugvolumens) auf Hypoxie und normoxische Hyperkapnie aufweisen [Trapp et al., J. Neurosci. 28, 8844-8850 (2008)]. Des Weiteren wurden TASK-1- und TASK-3-Kanäle in Motoneuronen des *Nervus hypoglossus,* dem XII. Hirnnerv, nachgewiesen, der eine wichtige Funktion für die Offenhaltung der oberen Atemwege hat [Berg et al., J. Neurosci. 24, 6693-6702 (2004)].

In einem Schlafapnoe-Modell am anästhesierten Schwein führte die nasale Gabe eines Kaliumkanal-Blockers, der im nanomolaren Bereich den TASK-1-Kanal blockiert, zu einer Hemmung der Kollapsibilität der pharyngealen Atemwegsmuskulatur und zu einer Sensibilisierung des negativen Druckreflexes der oberen Atemwege. Man vermutet, dass die nasale Gabe des Kaliumkanal-Blockers Mechanorezeptoren in den oberen Atemwegen depolarisiert und über Aktivierung des negativen Druckreflexes zu einer verstärkten Aktivität der Muskulatur der oberen Atemwege führt, wodurch eine Stabilisierung der oberen Atemwege erfolgt und ein Kollaps verhindert wird. Über eine solche Stabilisierung der oberen Atemwege kann die TASK-Kanal-Blockade für obstruktive Schlafapnoe und auch Schnarchen von großer Bedeutung sein [Wirth et al., Sleep 36, 699-708 (2013); Kiper et al., Pflugers Arch. 467, 1081-1090 (2015)].

Die obstruktive Schlafapnoe (OSA) ist eine schlafbedingte Atemstörung, die gekennzeichnet ist durch wiederholte Episoden der Obstruktion der oberen Atemwege. Bei der Einatmung wird die Durchgängigkeit der oberen Atemwege durch das Zusammenspiel zweier Gegenkräfte gewährleistet. Die dilatierenden Effekte der Muskulatur der oberen Atemwege wirken dem das Lumen verengenden negativen intraluminaren Druck entgegen. Die aktive Kontraktion des Zwerchfells und der anderen Atemhilfsmuskeln erzeugt einen Unterdruck in den Atemwegen und stellt so die treibende Kraft für die Atmung dar. Die Stabilität der oberen Atemwege wird maßgeblich von der Koordination und Kontraktionseigenschaft der dilatierenden Muskeln der oberen Atemwege bestimmt.

Der *Musculus genioglossus* spielt bei der Pathogenese der OSA eine entscheidende Rolle. Die Aktivität des *Musculus genioglossus* nimmt mit abnehmendem Druck im Pharynx im Sinne eines dilatierenden Kompensationsmechanismus zu. Innerviert vom *Nervus hypoglossus* zieht er die Zunge nach vorne und unten und erweitert auf diese Weise den pharyngealen Luftweg [Verse et al., Somnologie 3, 14-20 (1999)]. Die Anspannung der dilatierenden Muskeln der oberen Atemwege wird unter anderem über Mechanorezeptoren/Dehnungsrezeptoren im Nasen-Rachen-Raum moduliert [Bouillette et al., J. Appl. Physiol. Respir. Environ. Exerc. Physiol. 46, 772-779 (1979)]. Durch Lokalanästhesie des oberen Luftwegs kann bei Patienten mit schwerer Schlafapnoe im Schlaf eine zusätzliche Reduktion der Aktivität des *Musculus genioglossus* beobachtet werden [Berry et al., Am. J. Respir. Crit. Care Med. 156, 127-132 (1997)]. Patienten mit OSA haben eine hohe Mortalität und Morbidität infolge von Herz-Kreislauf-Erkrankungen wie z. B. Bluthochdruck, Herzinfarkt und Schlaganfall [Vrints et al., Acta Clin. Belg. 68, 169-178 (2013)].

Bei der zentralen Schlafapnoe kommt es infolge einer gestörten Hirnfunktion bzw. einer gestörten Atemregulation zu episodischen Hemmungen des Atemantriebs. Zentral bedingte Atemstörungen führen zu mechanischen Atemstillständen, d.h. es findet während dieser Episoden keine Atmungsaktivität statt, sämtliche Atemmuskeln einschließlich Zwerchfell stehen vorübergehend still. Bei der zentralen Schlafapnoe liegt keine Obstruktion der oberen Atemwege vor.

Beim primären Schnarchen kommt es ebenfalls nicht zu einer Obstruktion der oberen Atemwege. Durch die Verengung der oberen Atemwege erhöht sich allerdings die Strömungsgeschwindigkeit der ein- und ausgeatmeten Luft. Dies bewirkt im Verbund mit der erschlafften Muskulatur, dass die weichen Gewebe des Mund- und Rachenraumes im Luftstrom flattern. Dieses leichte Vibrieren erzeugt dann die typischen Schnarchgeräusche.

Das obstruktive Schnarchen (*upper airway resistance syndrome, heavy snoring,* Hypopnoe-Syndrom) wird durch eine wiederkehrende partielle Obstruktion der oberen Atemwege im Schlaf verursacht. Hierdurch kommt es zu einer Erhöhung des Atemwegswiderstands und somit zu einem Anstieg der Atemarbeit mit erheblichen intrathorakalen Druckschwankungen. Die negative intrathorakale Druckentwicklung während der Inspiration kann dabei Werte erreichen, wie sie infolge einer kompletten Atemwegsobstruktion bei der OSA auftreten. Die pathophysiologischen Auswirkungen auf Herz, Kreislauf und Schlafqualität entsprechen denen bei der obstruktiven Schlafapnoe. Die Pathogenese ist wie bei der OSA in einem gestörten Reflexmechanismus der Pharynx-dilatierenden Muskeln im Schlaf während der Inspiration anzunehmen. Das obstruktive Schnarchen stellt häufig die Vorstufe für die OSA dar *[*Hollandt et al., HNO 48, 628-634 (2000)].

Die derzeit verfügbaren Therapiemöglichkeiten von Schnarchen und von OSA sind begrenzt. Aus den 1980er Jahren sind Mischungen von oberflächenaktiven Substanzen bekannt, die den Widerstand der oberen Atemwege und das Schnarchen reduzieren sollen [Widdicombe and Davies, Eur Resp J 1, 785-791 (1988)]. Diese Mischungen enthalten NaCl, Glycerol, Polysorbat 80 und Benzalkoniumchlorid. Aus Versuchen an Hunden, denen diese Mischungen per Injektion in den Pharynx verabreicht wurden, wurde gefolgert, dass diese Mischungen den Widerstand der oberen Atemwege reduzieren, die Aktivität des *Musculus genioglossus* während der Einatmung und Ausatmung erhöhen und Schnarchgeräusche reduzieren. OSA wird in dem Artikel von Widdicombe nicht erwähnt und es wurde in diesem Modell auch nicht gezeigt, dass ein Kollaps der oberen Luftwege, der zu einer Apnoe führt, verhindert werden konnte. Das Modell von Widdicombe und Davies ist damit nicht prädiktiv für OSA.

Eine Zusammensetzung bestehend aus: 0.26% Glycerol, 0.2% Polysorbat 80, 0.9% Natriumchlorid und 0.15% Kaliumsorbat (ohne Benzalkoniumchlorid) ist als Asonor^{®} zur Therapie des Schnarchens auf dem Markt. In einer Studie des University State Hospital in Kopenhagen wurde die Wirksamkeit einer nasalen Gabe von Asonor^{®} im Vergleich zu "Asonor^{®}" ohne Polysorbat 80 im Hinblick auf eine Verbesserung des Schnarchens untersucht. Sowohl Asonor^{®} als auch "Asonor^{®}" ohne Polysorbat 80 bewirkten eine signifikante Verbesserung des Schnarchens [Report from the Departement of Neurology, University State Hospital, Copenhagen, Denmark. The effect of nasal application of Asonor^{®} and Polyglycoside 80 on snoring and sleep apnoea, 1989, http://www.chrapat.sk/img/klinicka-dokumentacia.pdf].

EP 2595685 B1 (U.S. Patent No. 9,132,243 B1) beansprucht ein pharmazeutisches Produkt umfassend einen Behälter, der eine flüssige Anti-Schnarch-Substanz enthält, wobei der Behälter einen Flüssigkeitsauslassabschnitt enthält, der konfiguriert ist, um die flüssige Anti-Schnarch-Substanz direkt in einen Nasengang in der Form eines Strahlstroms abzugeben. Die flüssige Anti-Schnarch-Substanz ist eine Anti-Schnarch-Lösung, die Natriumchlorid, Glycerol, Polysorbat und Natriumedetat und wahlweise Kaliumsorbat als Konservierungsstoff umfasst. Eine Therapie von Apnoe oder OSA ist in den ursprünglich eingereichten Anmeldeunterlagen von EP 2595685 B1 und U.S. Patent No. 9,132,243 B1 nicht offenbart. In der EP 2595685 B1 ist die beschriebene Anti-Schnarch-Substanz für eine Verwendung in der Behandlung von Schnarchen und Atemstillstand (Apnoe) beansprucht.

Für die Therapie der OSA ist derzeit keine pharmakologische Therapie verfügbar. Operationen und orale Vorrichtungen sind nur eingeschränkt wirksam. Behandlungsstandard ist die Therapie mit dem Continuous Positive Airway Pressure (CPAP) System. Die Rate der Einhaltung dieser Therapie liegt aber aufgrund der Unannehmlichkeit bei nur 50-70% und das System wird im Schnitt nicht mehr als 4 Stunden pro Nacht getragen.

Neue Substanzen, die als potente und selektive Inhibitoren von TASK-1- und/oder TASK-3-Kanälen agieren und sich als solche insbesondere zur Behandlung und/oder Prävention von Atemstörungen, einschließlich schlafbedingter Atemstörungen wie obstruktiven und zentralen Schlafapnoen und Schnarchen, sowie anderer Erkrankungen eignen, sind bekannt aus PCT/EP2016/079973 und PCT/EP2016/079544 (unveröffentlicht).

Die Wirkdauer der in der EP 15199270.8 und EP 15199268.2 offenbarten potenten und selektiven Inhibitoren von TASK-1- und/oder TASK-3-Kanälen ist bei nasaler Verabreichung nicht immer ausreichend, was eine Nachdosierung während der Nacht und damit eine Unterbrechung der Nachtruhe bzw. des Schlafs erforderlich macht.

Aufgabe der vorliegenden Erfindung ist es daher, eine wirkungsvolle pharmakologische Therapie zur Behandlung und/oder Prävention von Atemstörungen, einschließlich schlafbedingter Atemstörungen wie obstruktiven und zentralen Schlafapnoen und Schnarchen zur Verfügung zu stellen, die eine Alternative zur Behandlung mit dem CPAP System darstellt.

Eine weitere Aufgabe der vorliegenden Erfindung ist es, die Rate der Einhaltung einer Behandlung und/oder Prävention von Atemstörungen, einschließlich schlafbedingter Atemstörungen wie obstruktiven und zentralen Schlafapnoen und Schnarchen gegenüber dem derzeitigen Therapiestandard (Therapie von OSA: CPAP System) durch den Patienten zu erhöhen. Dazu sollte diese alternative Therapie einfach und bequem anwendbar sein und den Schlafenden nicht stören. Außerdem sollte diese alternative Therapie mit einer einmal täglichen Dosierung vor dem Schlafengehen eine ungestörte Nachtruhe ohne erneute Medikation ermöglichen.

Eine weitere Aufgabe der vorliegenden Erfindung ist es daher, die pharmakologisch wirksamen Substanzen zur Behandlung und/oder Prävention von Atemstörungen, einschließlich schlafbedingter Atemstörungen wie obstruktiven und zentralen Schlafapnoen und Schnarchen in einer Applikationsform zur Verfügung zu stellen, die für eine einmal tägliche nasale oder pharyngeale Gabe vor dem Schlafengehen geeignet ist. Insbesondere ist es eine Aufgabe der vorliegenden Erfindung, eine pharmakologisch wirksame Therapie zur Behandlung und/oder Prävention von Atemstörungen, einschließlich schlafbedingter Atemstörungen wie obstruktiven und zentralen Schlafapnoen und Schnarchen zur Verfügung zu stellen, die eine Wirkdauer von mindestens 4 Stunden aufweist.

Die Verlängerung der Wirkdauer von nasal verabreichten Wirkstoffen ist schwierig. Aufgrund physiologischer Gegebenheiten ist die Verweilzeit von Wirkstoffen, Partikeln, Kapseln und Ähnlichem an den Epithelzellen kurz. Das Epithel besteht zum Teil aus Flimmerzellen, welche haarförmige Strukturen, die Zilien aufweisen. Diese sind von einer Schleimschicht (Mukus) bedeckt, welche durch eine koordinierte Bewegung der Zilien in Richtung Rachen abtransportiert wird. Auf der Mukusschicht bleiben Fremdpartikel und Mikroorganismen nach nasaler Aufnahme haften und werden durch die mukoziliäre *Clearance* gemeinsam mit dem Mukus in Richtung Rachen und Oesophagus transportiert. Dadurch wirkt die mukoziliäre *Clearance* der nasalen Absorption von Wirkstoffen entgegen und stellt insbesondere eine Herausforderung für das Erreichen einer Wirkverlängerung dar. Die Flussrate des Mukus beträgt ungefähr 5 mm pro Minute, so dass sie alle 15-20 min erneuert wird. Für nasal applizierte Lösungen und Pulver wurden daher auch *Clearance*-Halbwertszeiten von 15 min bestimmt [Illum et al., Int J Pharm. 39, 189-199 (1987)], so dass Wirkstoffe prinzipiell nur kurz an der Mukosa verweilen, um eine Wirkung zu erzielen.

Eine Methode zur Erreichung einer Wirkverlängerung nach nasaler Applikation ist, die Kontaktzeit zwischen Wirkstoff und dem Absorptionsort in der Nase, den Epithelzellen, zu verlängern. Durch eine verlängerte Kontaktzeit wird die Absorption von Arzneistoffen in der Nase gesteigert. Die Wirkstoffaufnahme kann über einen längeren Zeitraum erfolgen, so dass zum einen eine verlängerte Wirkung bzw. Wirkdauer erreicht und zum anderen die insgesamt aufgenommene Arzneistoffmenge gesteigert werden kann. Methoden, um die Kontaktzeit zwischen dem Wirkstoff und den Epithelzellen zu erhöhen, sind unter anderem die Erhöhung der Viskosität, die Verwendung von bioadhäsiven Polymeren oder der Einsatz von Mikropartikeln.

Pennington et al. konnten bereits 1988 zeigen, dass durch die Erhöhung der Viskosität von nasal applizierten Lösungen mit Hydroxypropylmethylcellulose die *Clearance*-Rate reduziert wird [Pennington et al., Int J Pharm. 43, 221-224 (1988)]. Mit ansteigendem Polymeranteil und damit steigender Viskosität verlängerte sich die Halbwertszeit von 1 Stunde auf 2.2 Stunden. Verglichen mit den von Illum et al. [Illum et al., Int J Pharm. 39, 189-199 (1987)] beobachteten Halbwertszeiten für Lösungen von 15 min führte die Viskositätserhöhung somit zu einer deutlichen Verlängerung der Halbwertszeit. Viskose Lösungen und halbfeste Systeme wie Gele, Cremes und Salben lassen sich allerdings schwieriger applizieren als niedrig-viskose Formulierungen. Die Zerstäubung über ein Spray ist nicht mehr möglich und eine genaue Dosierung mit Hilfe von Applikatoren im Falle von halbfesten Systemen ist erschwert. Zudem können nasal applizierte halbfeste Systeme zu einer Blockade führen, die die nasale Atmung stören können. Neben der Verabreichung von höher-viskosen Lösungen und applikationsfertigen Gelen ist auch die Verabreichung von *in situ* Gelen denkbar [Majithiya et al., AAPS PharmSciTech 7 (3), Article 67 (2006)]. Hier wird die Gelierung erst innerhalb der Nase ausgelöst, z. B. durch eine Temperaturveränderung, eine Änderung des pH-Wertes oder die Anwesenheit von Ionen. Dadurch kann eine niedrig-viskose Lösung appliziert werden und die viskose Formulierung steht nach Gelierung am Depositionsort, der Nasenschleimhaut, mit ihren positiven Effekten zur Verfügung. Dadurch können Dosiersysteme für die Applikation verwendet werden, die eine genaue und einfache Applikation ermöglichen. Allerdings handelt es sich um komplexe und aufwändige Darreichungsformen, da die Gelbildung genau abgestimmt werden muss. Wird die Gelierung z. B. durch eine Temperaturänderung hervorgerufen, muss gewährleistet werden, dass die Gelierung erst bei physiologischen Temperaturen ausgelöst wird, bei der Lagerung allerdings noch unterbunden wird. Damit sind einerseits besondere Anforderungen an die Lagerung und Handhabung gestellt, um eine vorzeitige Gelierung zu vermeiden, andererseits ist der Entwicklungs- und Herstellungsaufwand eines solchen, sensitiven Systems sehr hoch.

Als bioadhäsive Polymere finden Stärke und Chitosan häufig Verwendung [Illum et al., J Controlled Release 87, 187-198 (2003)]. Chitosan ist ein bioadhäsives Polysaccharid und kann ausgeprägt mit den Epithelzellen und der Mukusschicht interagieren. Dadurch wird eine längere Kontaktzeit hervorgerufen, die für den Wirkstofftransport durch die Membran zur Verfügung steht. In der Literatur ist Chitosan weit verbreitet, allerdings handelt es sich hier vorwiegend um *in vitro* Versuche. Bisher ist Chitosan nicht für die nasale Applikation zugelassen (FDA Drug Databases, Inactive Ingredient Search for Approved Drug Products) und die potentielle Langzeit-Toxizität für eine chronische nasale Applikation ist nicht vollständig untersucht.

Eine weitere Möglichkeit, die Wirkung nach nasaler Wirkstoffverabreichung zu verlängern, stellt die Verkapselung des Wirkstoffs in polymere Mikropartikel dar [Cerchiara et al., Eur J Pharm Biopharm. 61, 195-200 (2005)]. Hierzu wird der Wirkstoff in einem geeigneten Polymer, das eine geringe Löslichkeit in Wasser aufweist, oder einer Polymerkombination, die zusätzlich noch eine Adhäsion der wirkstoffbeladenen Mikropartikel an die Nasenschleimhaut ermöglicht, eingebettet. Nach Einbringen dieser Darreichungsform in die Nase wird der Wirkstoff aus den Mikropartikeln je nach Eigenschaft des eingesetzten Polymers mittels Diffusion und/oder Polymerabbau/-erosion verzögert freigegeben, was zu einer verlängerten Wirkdauer des Wirkstoffs am Wirkort führt. Verfügt die verwendete Polymerkombination, aus der die Mikropartikel aufgebaut sind, zusätzlich noch über die Eigenschaft, an der Nasenschleimhaut anzuhaften, so ist mit einer verlängerten Verweildauer und damit Wirkdauer der nasal eingebrachten Medikation zu rechnen. Gerade die Kombination aus Mikropartikeln und bioadhäsiven Polymeren stellt daher einen viel beschriebenen Ansatz zur Verlängerung der Wirkdauer bei nasaler Applikation dar, da hier zwei Prinzipien - die verzögerte Freisetzung und die Erhöhung der Kontaktzeit - kombiniert werden. Dabei können die Mikropartikel direkt aus einem bioadhäsiven Polymer hergestellt werden [Illum et al., Int J Pharm. 39, 189-199 (1987)] oder es können andere Polymere wie Poly(lactid-co-glycolid (PLGA) für die Herstellung der Mikropartikel verwendet werden, die dann in einem weiteren Schritt mit dem bioadhäsiven Polymer überzogen werden [Pawar et al., Am Assoc Pharmac Sci J 12, 130-137 (2010)].

Neben der Verwendung der oben beschriebenen Mikropartikel kann eine Verlängerung der Wirkstofffreisetzung auch durch die Verwendung von suspendiertem statt gelöstem Wirkstoff erzeugt werden. Der eingesetzte Wirkstoff wird hierfür z. B. mikronisiert (Zerkleinerung zu Wirkstoffmikropartikeln) und in eine flüssige Phase eingearbeitet (suspendiert). Nach Applikation in die Nase, lösen sich die Wirkstoffpartikel am Wirkort verzögert auf. Erst der gelöste Wirkstoff kann über die Nasenschleimhaut absorbiert werden und dann wirksam werden. Die Auflösungskinetik, die über die Verlängerung des Wirkeffekts entscheidet, hängt u.a. von den physikochemischen Eigenschaften (z. B. Löslichkeit, Partikelgröße) des eingesetzten Wirkstoffs ab. Durch eine Verabreichung von Kristallsuspensionen von Glucocorticoiden konnte so beispielsweise eine lokale Wirkungsverlängerung erzielt werden [Rygg et al., Pharm Res. 33, 909-921(2016)].

Die Verarbeitung von Wirkstoffen in Kristallsuspensionen sowie die Verkapselung von Wirkstoffen in polymere Mikropartikel mit dem Ziel einer Wirkungsverlängerung nach nasaler Applikation ist mit zahlreichen Nachteilen verbunden.

Einerseits ist die Herstellung derartiger Darreichungsformen im Vergleich zu beispielsweise Wirkstofflösungen technisch um ein vielfaches komplexer. So erfordert die Herstellung von Kristallsuspensionen und polymeren Mikropartikeln eine Vielzahl von aufeinanderfolgenden Prozessschritten, die die Qualität der fertigen Darreichungsform signifikant beeinflussen. Die Funktionalität dieser komplexen Darreichungsformen kann aufgrund mangelnder Lagerstabilität ungünstig beeinflusst werden. So weisen Kristallsuspensionen beispielsweise Partikelsedimentation (inkl. Sedimentbildung) und/oder Veränderungen der Primärpartikelgröße während der Lagerung auf, was zu Inhomogenität innerhalb der Darreichungsform und damit Fehldosierungen führt.

Andererseits wird für die Herstellung von Kristallsuspensionen und polymeren Mikropartikeln der Einsatz zahlreicher Stabilisatoren und polymerer Matrixbildner benötigt, die nach nasaler Applikation zu lokalen Unverträglichkeiten/Reizungen führen können. So ist beispielsweise bekannt, dass zahlreiche Stabilisatoren zu einer unerwünschten Beeinflussung der Zilienbeweglichkeit, Zellyse und Inaktivierung von Enzymen führen können [Schinichiro et al., Int J Pharm. 9, 173-184 (1981)]. Während des hydrolytischen Abbaus von Polymeren wie etwa bioresorbierbaren Polyestern (z. B. PLGA), die häufig als Matrixbildner für Mikropartikel eingesetzt werden, kommt es zu einer Freisetzung von Abbauprodukten (z. B. Milch- und Glykolsäure), die den lokalen pH-Wert stark absenken können, wodurch es zu einer lokalen Irritation kommen kann. Lokale Irritationen können auch durch die Partikel selbst ausgelöst werden.

Darüber hinaus kann gerade die Verwendung von partikulären Systemen wie Kristallsuspensionen und polymeren Mikropartikeln, die mit einer verzögerten Freisetzung und Auflösung des Wirkstoffes einhergehen, dazu führen, dass bedingt durch die mukoziliäre Clearance ein nicht reproduzierbarer Anteil der Dosis vor der Absorption bereits als ungelöste Partikel abtransportiert und abgeschluckt wird. Das Verschlucken von Wirkstoff kann dann wiederrum zu einer großen Variabilität der Exposition führen [Malinovsky et al., Br J Anaesthesia 77, 203-207 (1996)].

Weiterhin ist der Einsatz von Kristallsuspensionen und polymeren Mikropartikeln mit komplexeren Anwendungshinweisen verbunden, die zu Anwendungsfehlern führen können, die wiederum den gewünschten Therapieerfolg gefährden.

Nachteile der beschriebenen Ansätze zur Verlängerung der Wirkung nasal verabreichter Wirkstoffe, wie viskose Systeme, Kristallsuspensionen und Mikropartikel, sind demnach der hohe Aufwand in der Herstellung, die Komplexität dieser Darreichungsformen, das Risiko für große Variabilität in der Exposition und nicht zuletzt die mangelnde Unbedenklichkeit der eingesetzten Hilfsstoffe (z. B. Polymere) für die nasale Applikation.

Überraschenderweise wurde in der vorliegenden Erfindung gezeigt, dass eine nasale Gabe einer Formulierung enthaltend eine therapeutisch wirksamen Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon in >2.5% bis 100% w/v Polyethylenglykol die Wirkdauer des Inhibitors des TASK-1 und/oder TASK-3 Kanals oder eines Hydrats, Solvats, Polymorphs oder Metaboliten hiervon oder eines pharmazeutisch verwendbaren Salzes hiervon in Abhängigkeit von deren Dosis deutlich verlängert.

Ein Gegenstand der vorliegenden Erfindung sind stabile pharmazeutische Formulierung zur nasalen oder pharyngealen Applikation enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon in >2.5% bis 100% w/v Polyethylenglykol und gegebenenfalls mindestens einen Hilfsstoff, wobei die Formulierung einen pH-Wert von 4 bis 8 aufweist.

Eine nasale oder pharyngeale Gabe einer therapeutisch wirksamen Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon in einer Formulierung enthaltend einen pH-Regulator und einen Lösungsvermittler ohne Zusatz von Polyethylenglykol führte auch bei Steigerung der Dosis des Inhibitors des TASK-1 und/oder TASK-3 Kanals nicht zu einer Verlängerung der Wirkdauer.

Überraschenderweise zeigten auch Formulierungen enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon und enthaltend 20% w/v Propylenglykol (statt Polyethylenglycol) und einen pH-Regulator und einen Lösungsvermittler keine Verlängerung der Wirkdauer des Inhibitors des TASK-1 und/oder TASK-3 Kanals.

Auch Formulierungen enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon und 1.25% w/v der Viskositäts-erhöhenden Substanz Na-Carboxymethylcellulose (Na-CMC) (statt PEG) und einen pH-Regulator und einen Lösungsvermittler zeigten keine Verlängerung der Wirkdauer des Inhibitors des TASK-1 und/oder TASK-3 Kanals, obwohl die Viskosität dieser Formulierung mit 1.25% w/v Na-CMC mit der Viskosität einer erfindungsgemäßen Formulierung enthaltend 20% w/v PEG400 vergleichbar ist. Dies weist darauf hin, dass eine durch die Zugabe von PEG bewirkte Viskositätserhöhung nicht der entscheidende Grund für die mit den erfindungsgemäßen Formulierungen beobachtete Verlängerung der Wirkdauer sein kann.

Auch eine Zusammensetzung enthaltend Polysorbat 80 und mindestens 5% w/v PEG400 in Phosphatpuffer, pH 7, ohne einen Inhibitor des TASK-1 und/oder TASK-3 Kanals zeigte in der vorliegenden Erfindung keine Wirkung.

Der Fachmann hatte keinen Ansatzpunkt dafür, die physikalische Therapie von OSA mittels CPAP zu ersetzen, da eine pharmakologische Alternative erstmals in der unveröffentlichten PCT/EP2016/079973 beschrieben ist. Auch gegen Schnarchen gibt es derzeit keine bzw. nur sehr begrenzte pharmakologische Therapien, so dass der Fachmann auch hier keinen Ansatzpunkt gehabt hätte, zur vorliegenden Erfindung zu gelangen. Auch wenn die in der PCT/EP2016/079973 beschriebenen TASK-1 und/oder TASK-3 Inhibitoren bekannt gewesen wäre, hätte der Fachmann keine Veranlassung gehabt zu vermuten, dass die geschilderte, sehr einfach handhabbare Lösung zur Verlängerung der Wirkdauer des Inhibitors des TASK-1 und/oder TASK-3 Kanals erfolgreich ist.

Im Stand der Technik gibt es keinen Hinweis darauf, dass eine Verlängerung der Wirkung von Inhibitoren des TASK-1 und/oder TASK-3 Kanals um mehrere Stunden im Hinblick auf OSA mit dem Einsatz des Standardformulierungshilfsstoffs Polyethylenglykol, aber nicht mit Propylenglykol, erreicht werden kann. Auch gibt es im Stand der Technik keinen Hinweis, dass eine Wirkverlängerung von mehreren Stunden ohne die Verwendung von im Stand der Technik zur Wirkverlängerung von nasal verabreichten Wirkstoffen beschriebenen, aufwändigen Ansätzen wie Mikropartikeln, Kristallsuspensionen oder bioadhäsive Systeme erreicht werden kann.

Darüber hinaus gibt es im Stand der Technik keinen Hinweis darauf, dass die Wirkverlängerung mit Hilfe der erfindungsgemäßen Formulierungen nur in einem spezifischen Konzentrationsbereich des Formulierungsbestandteils Polyethylenglykol erreicht werden kann. Auch ein Hinweis auf geeignete Konzentrationsbereiche der Formulierungsbestandteile ist dem Stand der Technik nicht zu entnehmen.

Im Rahmen der vorliegenden Erfindung wird die stabile pharmazeutische Formulierung nasal oder pharyngeal appliziert.

Im Rahmen der vorliegenden Erfindung werden die Begriffe "nasal" und "intranasal" synonym verwendet.

Im Rahmen der vorliegenden Erfindung sind stabile pharmazeutische Formulierungen, die für die nasale Applikation geeignet sind, Formulierungen in flüssiger, halbfester oder fester Form, beispielsweise Nasentropfen, Nasenlösungen, Nasengele, Nasensalben, Nasencremes oder pulverförmige Darreichungsformen.

Im Rahmen der vorliegenden Erfindung kann eine nasale Applikation beispielsweise mittels Nasenspray, Tropfpipette, Quetschflasche, COMOD^{®} System, Flüssigzerstäubern (z. B. piezoelektrischen Verneblern, Düsen- oder Ultraschall-Aerosolgeneratoren, Soft-Mist-Inhalern) oder Dosieraerosolen, bzw. Nasenapplikatoren für halbfeste Formulierungen (Tubenspitzen, Spatel) und/oder feste Formulierungen (Pulver) erfolgen. Gemäß einer Ausführungsform der vorliegenden Erfindung erfolgt die Applikation mittels Nasenspray.

Im Rahmen der vorliegenden Erfindung sind stabile pharmazeutische Formulierungen, die für die pharyngeale Applikation geeignet sind, Formulierungen in flüssiger, halbfester oder fester Form, beispielsweise Lösungen, Gele oder Pulver.

Im Rahmen der vorliegenden Erfindung kann eine pharyngeale Applikation mittels Inhalation unter Verwendung von Flüssigzerstäubern (z. B. piezoelektrischen Verneblern, Düsen- oder Ultraschall-Aerosolgeneratoren, Pumpsprays) oder Dosieraerosolen, oder mittels lokaler Applikation unter Verwendung eines Bronchoskops (Instillation), einer Tropfpipette, Quetschflasche oder ähnlichem erfolgen.

Die therapeutische Wirkung im Sinne der vorliegenden Erfindung ist definiert als eine Reduktion des Apnoe-Hypopnoe Index (AHI) eines Patienten mit schlafbedingten Atemstörungen wie obstruktiven und zentralen Schlafapnoen und Schnarchen nach nasaler oder pharyngealer Gabe einer erfindungsgemäßen Formulierung enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon.

Gemäß einer Ausführungsform der vorliegenden Erfindung ist die therapeutische Wirkung definiert als eine Reduktion des Apnoe-Hypopnoe Index (AHI) eines Patienten mit schlafbedingten Atemstörungen wie obstruktiven und zentralen Schlafapnoen und Schnarchen nach nasaler oder pharyngealer Gabe einer erfindungsgemäßen Formulierung enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon um mindestens 20%.

Gemäß einer Ausführungsform der vorliegenden Erfindung ist die therapeutische Wirkung definiert als eine Reduktion des Apnoe-Hypopnoe Index (AHI) eines Patienten mit schlafbedingten Atemstörungen wie obstruktiven und zentralen Schlafapnoen und Schnarchen nach nasaler oder pharyngealer Gabe einer erfindungsgemäßen Formulierung enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon um mindestens 20%, mindestens 25%, mindestens 30%, mindestens 35%, mindestens 40%, mindestens 45%, mindestens 50%, mindestens 55%, mindestens 60%, mindestens 65%, mindestens 70%, mindestens 75% oder mindestens 80%.

Die Wirkdauer im Sinne der vorliegenden Erfindung ist definiert als die Zeit nach nasaler oder pharyngealer Gabe einer erfindungsgemäßen Formulierung enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon an einen Patienten mit schlafbedingten Atemstörungen wie obstruktiven und zentralen Schlafapnoen und Schnarchen, in der der Apnoe-Hypopnoe Index (AHI) dieses Patienten reduziert wird.

Gemäß einer Ausführungsform der vorliegenden Erfindung ist die Wirkdauer definiert als die Zeit nach nasaler oder pharyngealer Gabe einer erfindungsgemäßen Formulierung enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon an einen Patienten mit schlafbedingten Atemstörungen wie obstruktiven und zentralen Schlafapnoen und Schnarchen, in der der Apnoe-Hypopnoe Index (AHI) dieses Patienten um mindestens 20% reduziert wird.

Gemäß einer Ausführungsform der vorliegenden Erfindung ist die Wirkdauer definiert als die Zeit nach nasaler oder pharyngealer Gabe einer erfindungsgemäßen Formulierung enthaltend eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon an einen Patienten mit schlafbedingten Atemstörungen wie obstruktiven und zentralen Schlafapnoen und Schnarchen, in der der Apnoe-Hypopnoe Index (AHI) dieses Patienten um mindestens 20%, mindestens 25%, mindestens 30%, mindestens 35%, mindestens 40%, mindestens 45%, mindestens 50%, mindestens 55%, mindestens 60%, mindestens 65%, mindestens 70%, mindestens 75% oder mindestens 80% reduziert wird.

Im Sinne der vorliegenden Erfindung beträgt die Wirkdauer mindestens 3 Stunden oder mindestens 3.5 Stunden oder mindestens 4 Stunden oder mindestens 4.5 Stunden oder mindestens 5 Stunden oder mindestens 5.5 Stunden oder mindestens 6 Stunden oder mindestens 6.5 Stunden oder mindestens 7 Stunden oder mindestens 7.5 Stunden oder mindestens 8 Stunden. Gemäß einer Ausführungsform der vorliegenden Erfindung beträgt die Wirkdauer mindestens 3 Stunden. Gemäß einer Ausführungsform der vorliegenden Erfindung beträgt die Wirkdauer mindestens 4 Stunden. Gemäß einer Ausführungsform der vorliegenden Erfindung beträgt die Wirkdauer mindestens 5 Stunden.

Eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon im Sinne der vorliegenden Erfindung ist definiert als die Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon, die bei nasaler oder pharyngealer Applikation eine Wirkdauer von mindestens 3 Stunden oder mindestens 3.5 Stunden oder mindestens 4 Stunden oder mindestens 4.5 Stunden oder mindestens 5 Stunden oder mindestens 5.5 Stunden oder mindestens 6 Stunden oder mindestens 6.5 Stunden oder mindestens 7 Stunden oder mindestens 7.5 Stunden oder mindestens 8 Stunden zeigt.

Eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon im Sinne der vorliegenden Erfindung ist definiert als die Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon, die bei nasaler oder pharyngealer Applikation eine Wirkdauer von mindestens 3 Stunden zeigt.

Eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon im Sinne der vorliegenden Erfindung ist definiert als die Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon, die bei nasaler oder pharyngealer Applikation eine Wirkdauer von mindestens 4 Stunden zeigt.

Eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon im Sinne der vorliegenden Erfindung ist definiert als die Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon, die bei nasaler oder pharyngealer Applikation eine Wirkdauer von mindestens 5 Stunden zeigt.

Im Rahmen der vorliegenden Erfindung sind Hilfsstoffe Stoffe, die in der stabilen pharmazeutischen Formulierung zum Beispiel dazu dienen, den pH-Wert einzustellen oder zu stabilisieren, die Löslichkeit des Wirkstoffs zu erhöhen, die Zubereitung mikrobiologisch und physikalisch zu stabilisieren, die Viskosität der Formulierung zu verändern oder den Geschmack oder das Aussehen zu verbessern.

Beispiele für Hilfsstoffe im Sinne der vorliegenden Erfindung sind pH-Regulatoren, Lösungsvermittler, Antioxidantien, Stabilisatoren, Verdickungsmittel, Konservierungsmittel, Tonizitäts-einstellende Substanzen, Aromen, Duftstoffe oder Farbstoffe.

Gegenstand der vorliegenden Erfindung sind auch erfindungsgemäße stabile pharmazeutische Formulierungen zur nasalen oder pharyngealen Applikation, wobei der gegebenenfalls mindestens eine Hilfsstoff ausgewählt ist aus der Gruppe bestehend aus mindestens einem pH-Regulator, mindestens einem Lösungsvermittler, mindestens einem Antioxidans, mindestens einem Stabilisator, mindestens einem Verdickungsmittel, mindestens einem Konservierungsmittel, mindestens einer Tonizitätseinstellenden Substanz, mindestens einem Aroma, mindestens einem Duftstoff, und mindestens einem Farbstoff.

pH-Regulatoren im Sinne der vorliegenden Erfindung sind beispielsweise Puffer, wie Citronensäure und ihre Salze, Essigsäure und ihre Salze und Phosphorsäure und ihre Salze, oder anorganische Säuren wie beispielsweise Salzsäure, Borsäure, Carbonsäuren, Bicarbonsäuren, Aminosäuren oder organische Säuren wie beispielsweise Monocarbonsäuren wie Oxocarbonsäuren oder Polycarbonsäuren, oder Basen wie beispielsweise Natriumhydroxid, Kaliumhydroxid, Natriumcarbonat, Natriumhydrogencarbonat.

Gegenstand der vorliegenden Erfindung sind auch erfindungsgemäße stabile pharmazeutische Formulierungen zur nasalen oder pharyngealen Applikation, wobei der gegebenenfalls mindestens eine pH-Regulator ausgewählt ist aus der Gruppe bestehend aus Citronensäure und ihren Salzen, Essigsäure und ihren Salzen, Phosphorsäure und ihren Salzen, Salzsäure, Borsäure, Carbonsäuren, Bicarbonsäuren, Aminosäuren, Oxocarbonsäuren, Polycarbonsäuren, Natriumhydroxid, Kaliumhydroxid, Natriumcarbonat, und Natriumhydrogencarbonat.

Gemäß einer Ausführungsform der Erfindung ist der pH-Regulator ein Phosphatpuffer. Gemäß einer Ausführungsform der Erfindung ist der pH-Regulator ein Phosphatpuffer, der die Lösung im Sinne der vorliegenden Erfindung auf einen pH-Wert zwischen 4 und 8 puffert. Der bevorzugte pH-Bereich ist zwischen 7 und 8. Gemäß einer Ausführungsform beträgt der pH-Wert der erfindungsgemäßen Formulierungen 7.

Lösungsvermittler im Sinne der vorliegenden Erfindung sind beispielsweise Komplexbildner (beispielsweise Cyclodextrine und Natrium EDTA (Natriumethylendiamintetraessigsäure)), Cosolventien (beispielsweise Ethanol, Propylenglykol, Dimethylacetamid,) und Tenside. In die Gruppe der Tenside fallen beispielsweise Fettalkohole (beispielsweise Cetylalkohol), Phospholipide (beispielsweise Lecithin), Sterole (beispielsweise Cholesterol), Gallensäuresalze, Saponine, Glycerolfettsäureester (beispielsweise Glycerolmonostearat), Polyoxyethylen-fettsäureester (beispielsweise Polyoxyethylenstearat), Polyoxyethylensorbitanfettsäureester (wie Tween^{®}, beispielsweise Polysorbat 20 (Polyoxyethylen-(20)-sorbitanmonolaurat), Polysorbat 21 (Polyoxyethylen-(4)-sorbitanmonolaurat), Polysorbat 40 (Polyoxyethylen-(20)-sorbitanmonopalmitat), Polysorbat 60 (Polyoxyethylen-(20)-sorbitanmonostearat), Polysorbat 61 (Polyoxyethylen-(4)-sorbitanmonostearat), Polysorbat 65 (Polyoxyethylen-(20)-sorbitantristearat), Polysorbat 80 (Polyoxyethylen-(20)-sorbitanmonooleat), Polysorbat 81 (Polyoxyethylen-(5)-sorbitanmonooleat), Polysorbat 85 (Polyoxyethylen-(20)-sorbitantrioleat), Polysorbat 120 (Polyoxyethylen-(20)-sorbitanmonoisostearat)), Sorbitanfettsäureester (wie Span^{®}, beispielsweise Sorbitanmonolaurat (Span^{®} 20), Sorbitanmonopalmitat (Span^{®} 40), Sorbitanmonostearat (Span^{®} 60) Sorbitantristearat (Span^{®} 65) Sorbitanmonooleat (Span^{®} 80), Sorbitansesquioleat (Span^{®} 83), Sorbitantrioleat (Span^{®} 85), Polyoxyethylenglycerolfettsäureester (beispielsweise Polyoxyethylenglycerolmonostearat, Polyoxyethylenglycerolricinoleat, Polyoxyethylen-glyceroltriricinoleat), Polyoxyethylenfettalkoholether (beispielsweise Polyoxyethylenlaurylether, Polyoxyethylencetylstearylether), Polyoxypropylen-Polyoxyethylen-Blockcopolymere (beispielsweise Poloxamer), Alkylsulfate (beispielsweise Natriumlaurylsulfat, Natriumcetylstearylsulfat), Alkaliseifen (beispielsweise Natriumpalmitat, Natriumstearat) und Saccharose-Fettsäureester. Gemäß einer Ausführungsform der Erfindung ist der Lösungsvermittler ausgewählt aus der Gruppe bestehend aus Ethanol, Polysorbat 20, Polyoxyethylen(8)-stearat und Polysorbat 80. Gemäß einer Ausführungsform der Erfindung ist der Lösungsvermittler Polysorbat 80.

Gegenstand der vorliegenden Erfindung sind auch erfindungsgemäße stabile pharmazeutische Formulierungen zur nasalen oder pharyngealen Applikation, wobei der gegebenenfalls mindestens eine Lösungsvermittler ausgewählt ist aus der Gruppe bestehend aus Ethanol, Polysorbat 20, Polyoxyethylen(8)-stearat und Polysorbat 80.

Sofern als Lösungsvermittler ein Tensid in den erfindungsgemäßen Formulierungen enthalten ist, beträgt die Konzentration dieses Tensids minimal seine kritische Mizellenbildungskonzentration (CMC, critical micelle concentration) und maximal die für die nasale oder pharyngeale Applikation maximal zugelassene Menge. Die CMC von Polysorbat 80 beträgt 0.001% w/v, die maximal pharmazeutisch zugelassene Konzentration beträgt 10% w/v. Bei Verwendung von Polysorbat 80 als Lösungsvermittler ist Polysorbat 80 in einer Konzentration von 0.001-10% w/v, oder 0.1-10% w/v, oder 1-10% w/v oder 5-10% w/v in den erfindungsgemäßen Formulierungen enthalten. Alternativ kann Polysorbat 80 auch in Konzentrationen bis 15% w/v oder bis 20% w/v in den erfindungsgemäßen Formulierungen enthalten sein.

Antioxidantien im Sinne der vorliegenden Erfindung sind beispielsweise Citronensäure, Butylhydroxyanisol, Butylhydroxytoluol, EDTA, eine Begasung mit Stickstoff, Tocopherol, Ascorbinsäure, Glutathion, Cystein, Sulfite (beispielsweise Natriumsulfit, Natriumhydrogensulfit), Disulfite (beispielsweise Natriumpyrosulfit), Ascorbinsäureester oder Gallate. Gemäß einer Ausführungsform der Erfindung ist das Antioxidans ausgewählt aus der Gruppe bestehend aus Citronensäure, Butylhydroxyanisol, Butylhydroxytoluol, EDTA und einer Begasung mit Stickstoff. Gemäß einer Ausführungsform der Erfindung ist das Antioxidans Butylhydroxyanisol.

Gegenstand der vorliegenden Erfindung sind auch erfindungsgemäße stabile pharmazeutische Formulierungen zur nasalen oder pharyngealen Applikation, wobei das gegebenenfalls mindestens eine Antioxidans ausgewählt ist aus der Gruppe bestehend aus Citronensäure, Butylhydroxyanisol, Butylhydroxytoluol, EDTA und einer Begasung mit Stickstoff.

Eine Ausführungsform der vorliegenden Erfindung betrifft stabile pharmazeutische Formulierungen zur nasalen oder pharyngealen Applikation enthaltend:
eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon in >2.5% bis 100% w/v Polyethylenglykol und ein Antioxidans und gegebenenfalls mindestens einen weiteren Hilfsstoff, wobei die Formulierung einen pH-Wert von 4 bis 8 aufweist.

Konservierungsmittel im Sinne der vorliegenden Erfindung sind beispielsweise phenolische Substanzen wie Phenol oder Kresol, Alkohole wie Ethanol, Chlorbutanol, Phenylethanol, oder Propylenglycol, Invertseifen wie Benzalkoniumchlorid oder Benzethoniumchlorid, Benzoesäure und deren Salze, Sorbinsäure und deren Salze, Dehydroessigsäure und Schwefelsäure und deren Salze, Natriumhydrogensulfit, Parabene, einschließlich Methylparaben und Propylparaben oder Thiomersal. Gemäß einer Ausführungsform der Erfindung ist das Konservierungsmittel ausgewählt aus der Gruppe bestehend aus C₈-C₁₈ Alkoniumchlorid, Methylparaben, Propylparaben, Sorbinsäure, Chlorbutanol und Benzalkoniumchlorid. Gemäß einer Ausführungsform der Erfindung ist das Konservierungsmittel Benzalkoniumchlorid.

Gegenstand der vorliegenden Erfindung sind auch erfindungsgemäße stabile pharmazeutische Formulierungen zur nasalen oder pharyngealen Applikation, wobei das gegebenenfalls mindestens eine Konservierungsmittel ausgewählt ist aus der Gruppe bestehend aus C₈-C₁₈ Alkoniumchlorid, Methylparaben, Propylparaben, Sorbinsäure, Chlorbutanol und Benzalkoniumchlorid.

Tonizitäts-einstellende Substanzen im Sinne der vorliegenden Erfindung sind beispielsweise Salze (z. B. von Plasmakationen mit physiologisch tolerierbaren Gegenionen), Zucker (z. B. Glucose, Saccharose), Zuckeralkohole (z. B. Mannitol, Sorbitol), Glykole (z. B. Propylenglykole) und andere nichtionische Polyolmaterialien.

Verdickungsmittel im Sinne der vorliegenden Erfindung sind beispielsweise natürliche Gummis, Alginsäure, Pektine, Stärke und Stärkederivate, Gelatine, Poloxamere (Blockcopolymere Ethylenoxid und Propylenoxid) Zellulosederivate, Acrylsäurepolymere, oder Vinylpolymere.

Gemäß einer Ausführungsform der vorliegenden Erfindung enthalten die erfindungsgemäßen Formulierungen als Hilfsstoffe mindestens einen pH-Regulator. Gemäß einer Ausführungsform der vorliegenden Erfindung enthalten die erfindungsgemäßen Formulierungen als Hilfsstoffe mindestens ein Antioxidans. Gemäß einer Ausführungsform der vorliegenden Erfindung enthalten die erfindungsgemäßen Formulierungen als Hilfsstoffe mindestens einen Lösungsvermittler. Gemäß einer Ausführungsform der vorliegenden Erfindung enthalten die erfindungsgemäßen Formulierungen als Hilfsstoffe mindestens einen pH-Regulator und mindestens einen Lösungsvermittler. Gemäß einer Ausführungsform der vorliegenden Erfindung enthalten die erfindungsgemäßen Formulierungen als Hilfsstoffe mindestens ein Antioxidans und mindestens einen Lösungsvermittler. Gemäß einer Ausführungsform der vorliegenden Erfindung enthalten die erfindungsgemäßen Formulierungen als Hilfsstoffe mindestens einen pH-Regulator, mindestens einen Lösungsvermittler und mindestens ein Antioxidans. Gemäß einer Ausführungsform der vorliegenden Erfindung enthalten die erfindungsgemäßen Formulierungen als Hilfsstoffe mindestens einen pH-Regulator, mindestens einen Lösungsvermittler, mindestens ein Antioxidans und mindestens einen Konservierungsstoff.

Gegenstand der vorliegenden Erfindung sind auch erfindungsgemäße stabile pharmazeutische Formulierungen zur nasalen oder pharyngealen Applikation, wobei die Formulierung 5 bis 100% w/v Polyethylenglykol 400, 0 bis 10% w/v eines Lösungsvermittlers, 0 bis 95% w/v eines pH-Regulators und gegebenenfalls mindestens einen weiteren Hilfsstoff enthält.

Polyethylenglykol im Sinne der vorliegenden Erfindung ist definiert als ein Polyethylenglykol der allgemeinen Formel (II) 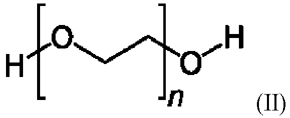 mit einer molaren Masse von 44 g/mol pro Wiederholungseinheit und zusätzlich dem Gewicht des eingelagerten Wassers. Die Summenformel von Polyethylenglykol ist C₂ₙH₄ₙ₊₂Oₙ₊₁.

Im Rahmen der vorliegenden Erfindung sind Polyethylenglykole mit einer mittleren molaren Masse von 200 bis 3,350 Da verwendbar. Gemäß einer weiteren Ausführungsform werden in der vorliegenden Erfindung Polyethylenglykole mit einem Molekulargewicht von 200 bis 600 oder 300 bis 400 oder 400 verwendet. Diese sind für die nasale Applikation zugelassen.

Eine Ausführungsform der vorliegenden Erfindung ist eine erfindungsgemäße stabile pharmazeutische Formulierung zur nasalen oder pharyngealen Applikation, wobei die Formulierung >2.5 w/v bis 100% w/v Polyethylenglykol mit einem Molekulargewicht von 200 bis 600 enthält und gegebenenfalls mindestens einen pH-Regulator und gegebenenfalls mindestens einen Lösungsvermittler und gegebenenfalls mindestens einen weiteren Hilfsstoff enthält.

PEG 400 weist eine mittlere molare Masse von 400 g/mol auf, PEG 300 weist eine mittlere molare Masse von 300 g/mol auf.

Im Rahmen der vorliegenden Erfindung liegt die dynamische Viskosität (bei 20°C) der erfindungsgemäßen Formulierungen zwischen 0.5 und 1480 mPa*s, bevorzugt zwischen 1.0 und 140 mPa*s. Erfindungsgemäße Formulierungen für die nasale Applikation mittels Nasenspray haben eine bevorzugte dynamische Viskosität (bei 20°C) zwischen 1.0 und 140 mPa*s. Erfindungsgemäße Formulierungen für die nasale Applikation mittels Nasentropfen haben eine bevorzugte dynamische Viskosität (bei 20°C) zwischen 1.0 und 1480 mPa*s.

Eine Ausführungsform der vorliegenden Erfindung ist eine erfindungsgemäße stabile pharmazeutische Formulierung zur nasalen oder pharyngealen Applikation, wobei die Formulierung eine Viskosität bei 20 °C von 0.5 - 200 mPa*s, bevorzugt 1 - 20 mPa*s aufweist.

Eine erfindungsgemäße Formulierung enthaltend 20% w/v PEG400 und 10% w/v Polysorbat 80 in Phosphatpuffer hat eine dynamischer Viskosität von ca. 6 mPa*s.

Im Rahmen der vorliegenden Erfindung liegt die bevorzugte Tröpfchengröße (angegeben als medianer Volumendurchmesser) bei einer zerstäubten Formulierung zwischen 5 und 300 µm, bevorzugt zwischen 30 und 100 µm. Dies ist unabhängig davon, ob die Gabe nasal oder pharyngeal erfolgt.

Eine Ausführungsform der vorliegenden Erfindung ist eine erfindungsgemäße stabile pharmazeutische Formulierung zur nasalen oder pharyngealen Applikation, wobei die Formulierung als Nasenspray verabreicht wird und eine Tröpfchengröße als medianer Volumendurchmesser von 5 - 300 µm, bevorzugt 30 - 100 µm aufweist.

Gemäß einer Ausführungsform der vorliegenden Erfindung enthalten die erfindungsmäßen Formulierungen > 2.5% w/v bis 100% w/v oder 3% w/v bis 100% w/v oder 3% w/v bis 90% w/v oder 3% w/v bis 80% w/v oder 3% w/v bis 70% w/v oder 3% w/v bis 60% w/v oder 3% w/v bis 50% w/v oder 3% w/v bis 40% w/v oder 3% w/v bis 30% w/v oder 3% w/v bis 20% w/v oder 3% w/v bis 10% w/v oder 3% w/v bis 5% w/v oder 5% w/v bis 100% w/v oder 5% w/v bis 90% w/v oder 5% w/v bis 80% w/v oder 5% w/v bis 70% w/v oder 5% w/v bis 60% w/v oder 5% w/v bis 50% w/v oder 5% w/v bis 40% w/v oder 5% w/v bis 50% w/v oder 5% w/v bis 20% w/v oder 5% w/v bis 10% w/v oder 5% w/v PEG200 oder PEG300 oder PEG400 oder PEG500 oder PEG600.

Gemäß einer Ausführungsform der vorliegenden Erfindung enthalten die erfindungsmäßen Formulierungen > 2.5% w/v bis 100% w/v oder 3% w/v bis 100% w/v oder 3% w/v bis 90% w/v oder 3% w/v bis 80% w/v oder 3% w/v bis 70% w/v oder 3% w/v bis 60% w/v oder 3% w/v bis 50% w/v oder 3% w/v bis 40% w/v oder 3% w/v bis 30% w/v oder 3% w/v bis 20% w/v oder 3% w/v bis 10% w/v oder 3% w/v bis 5% w/v oder 5% w/v bis 100% w/v oder 5% w/v bis 90% w/v oder 5% w/v bis 80% w/v oder 5% w/v bis 70% w/v oder 5% w/v bis 60% w/v oder 5% w/v bis 50% w/v oder 5% w/v bis 40% w/v oder 5% w/v bis 50% w/v oder 5% w/v bis 20% w/v oder 5% w/v bis 10% w/v oder 5% w/v PEG400.

Gemäß einer Ausführungsform der vorliegenden Erfindung enthalten die erfindungsmäßen Formulierungen den Inhibitor des TASK-1 und/oder TASK-3 Kanals in > 2.5 bis 100% w/v PEG400. Gemäß einer Ausführungsform der vorliegenden Erfindung enthalten die erfindungsmäßen Formulierungen den Inhibitor des TASK-1 und/oder TASK-3 Kanals in 5 bis 100% w/v PEG400. Gemäß einer Ausführungsform der vorliegenden Erfindung enthalten die erfindungsmäßen Formulierungen den Inhibitor des TASK-1 und/oder TASK-3 Kanals in 10 bis 100% w/v PEG400. Gemäß einer Ausführungsform der vorliegenden Erfindung enthalten die erfindungsmäßen Formulierungen den Inhibitor des TASK-1 und/oder TASK-3 Kanals in 20 bis 100% w/v PEG400. Gemäß einer Ausführungsform der vorliegenden Erfindung enthalten die erfindungsmäßen Formulierungen den Inhibitor des TASK-1 und/oder TASK-3 Kanals in 5 bis 20% w/v PEG400. Gemäß einer Ausführungsform der vorliegenden Erfindung enthalten die erfindungsmäßen Formulierungen den Inhibitor des TASK-1 und/oder TASK-3 Kanals in 20% w/v PEG400.

Im Rahmen der vorliegenden Erfindung ist ein Wirkstoff definiert als ein Inhibitor des TASK-1 und/oder TASK-3 Kanals oder ein Hydrat, Solvat, Polymorph, oder Metabolit hiervon oder ein pharmazeutisch verwendbares Salzes hiervon.

Erfindungsgemäße stabile pharmazeutische Formulierung sind beispielsweise solche Formulierungen, in denen der mindestens eine Inhibitor des TASK-1 und/oder TASK-3 Kanals ausgewählt ist aus den in der PCT/EP2016/079973 beschriebenen Verbindungen.

Erfindungsgemäße stabile pharmazeutische Formulierung sind beispielsweise solche Formulierungen, in denen der mindestens eine Inhibitor des TASK-1 und/oder TASK-3 Kanals ausgewählt ist aus Verbindungen der allgemeinen Formel (I), 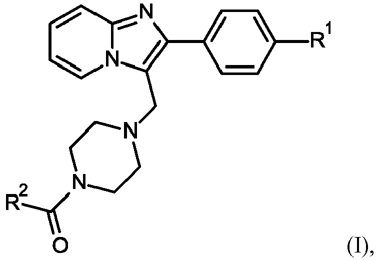 in welcher
- R¹: für Halogen, Cyano, (C₁-C₄)-Alkyl, Cyclopropyl oder Cyclobutyl steht
und
- R²: für (C₄-C₆)-Cycloalkyl steht, worin eine Ring-CH₂-Gruppe gegen -O- ausgetauscht sein kann,
oder
für eine Phenyl-Gruppe der Formel (a) oder eine Pyridyl-Gruppe der Formel (b) 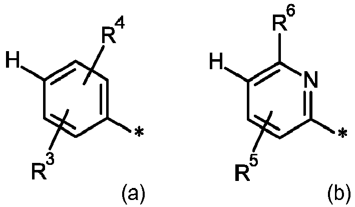 steht,
worin * die Verknüpfung zur angrenzenden Carbonyl-Gruppe markiert und
- R³: Fluor, Chlor, Brom, Cyano, (C₁-C₃)-Alkyl oder (C₁-C₃)-Alkoxy bedeutet,
wobei (C₁-C₃)-Alkyl und (C₁-C₃)-Alkoxy bis zu dreifach mit Fluor substituiert sein können,
- R⁴: Wasserstoff, Fluor, Chlor, Brom oder Methyl bedeutet,
- R⁵: Wasserstoff, Fluor, Chlor, Brom oder Methyl bedeutet
und
R⁶ Wasserstoff, (C₁-C₃)-Alkoxy, Cyclobutyloxy, Oxetan-3-yloxy, Tetrahydrofuran-3-yloxy oder Tetrahydro-2*H*-pyran-4-yloxy bedeutet,
wobei (C₁-C₃)-Alkoxy bis zu dreifach mit Fluor substituiert sein kann,
sowie ihre Salze, Solvate und Solvate der Salze.

Erfindungsgemäße stabile pharmazeutische Formulierung sind beispielsweise solche Formulierungen, in denen der mindestens eine Inhibitor des TASK-1 und/oder TASK-3 Kanals ausgewählt ist aus Verbindungen der oben angegebenen Formel (I), worin
- R¹: für Fluor, Chlor, Brom, Methyl, Isopropyl, *tert*.-Butyl oder Cyclopropyl steht
und
- R²: für Cyclobutyl, Cyclopentyl oder Cyclohexyl steht
oder
für eine Phenyl-Gruppe der Formel (a) oder eine Pyridyl-Gruppe der Formel (b) 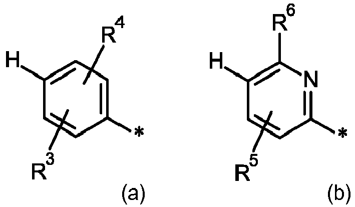 steht,
worin * die Verknüpfung zur angrenzenden Carbonyl-Gruppe markiert und
- R³: Fluor, Chlor, Cyano, (C₁-C₃)-Alkyl, (C₁-C₃)-Alkoxy oder Trifluormethoxy bedeutet,
- R⁴: Wasserstoff, Fluor oder Chlor bedeutet,
- R⁵: Wasserstoff, Fluor, Chlor, Brom oder Methyl bedeutet
und
- R⁶: Wasserstoff oder (C₁-C₃)-Alkoxy, das bis zu dreifach mit Fluor substituiert sein kann, bedeutet,
sowie ihre Salze, Solvate und Solvate der Salze.

Erfindungsgemäße stabile pharmazeutische Formulierung sind beispielsweise solche Formulierungen, in denen der mindestens eine Inhibitor des TASK-1 und/oder TASK-3 Kanals ausgewählt ist aus Verbindungen der Formel (I), worin
- R¹: für Chlor oder Brom steht,
sowie ihre Salze, Solvate und Solvate der Salze.

Erfindungsgemäße stabile pharmazeutische Formulierung sind beispielsweise solche Formulierungen, in denen der mindestens eine Inhibitor des TASK-1 und/oder TASK-3 Kanals ausgewählt ist aus Verbindungen der Formel (I), worin
- R¹: für Methyl, Isopropyl, *tert.-Butyl* oder Cyclopropyl steht,
sowie ihre Salze, Solvate und Solvate der Salze.

Erfindungsgemäße stabile pharmazeutische Formulierung sind auch solche Formulierungen, in denen der mindestens eine Inhibitor des TASK-1 und/oder TASK-3 Kanals ausgewählt ist aus Verbindungen der Formel (I), worin
- R²: für Cyclobutyl, Cyclopentyl oder Cyclohexyl steht,
sowie ihre Salze, Solvate und Solvate der Salze.

Erfindungsgemäße stabile pharmazeutische Formulierung sind auch solche Formulierungen, in denen der mindestens eine Inhibitor des TASK-1 und/oder TASK-3 Kanals ausgewählt ist aus Verbindungen der Formel (I), worin
- R²: für eine Phenyl-Gruppe der Formel (a) 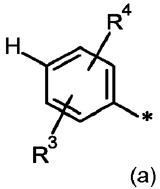
steht,
worin * die Verknüpfung zur angrenzenden Carbonyl-Gruppe markiert,
- R³: Fluor, Chlor, Cyano, (C₁-C₃)-Alkyl oder (C₁-C₃)-Alkoxy bedeutet
und
- R⁴: Wasserstoff, Fluor oder Chlor bedeutet,
sowie ihre Salze, Solvate und Solvate der Salze.

Erfindungsgemäße stabile pharmazeutische Formulierung sind auch solche Formulierungen, in denen der mindestens eine Inhibitor des TASK-1 und/oder TASK-3 Kanals ausgewählt ist aus Verbindungen der Formel (I), worin
- R²: für eine Pyridyl-Gruppe der Formel (b) 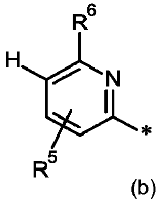
steht,
worin * die Verknüpfung zur angrenzenden Carbonyl-Gruppe markiert,
- R⁵: Wasserstoff, Chlor oder Brom bedeutet
und
- R⁶: (C₁-C₃)-Alkoxy, das bis zu dreifach mit Fluor substituiert sein kann, bedeutet,
sowie ihre Salze, Solvate und Solvate der Salze.

Erfindungsgemäße stabile pharmazeutische Formulierung sind auch solche Formulierungen, in denen der mindestens eine Inhibitor des TASK-1 und/oder TASK-3 Kanals ausgewählt ist aus Verbindungen der Formel (I), worin
- R¹: für Chlor, Brom, Isopropyl oder Cyclopropyl steht
und
- R²: für Cyclobutyl, Cyclopentyl oder Cyclohexyl steht
oder
für eine Phenyl-Gruppe der Formel (a) oder eine Pyridyl-Gruppe der Formel (b) 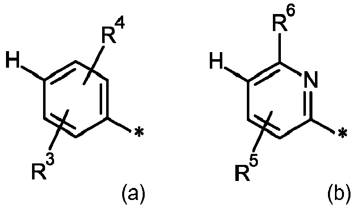 steht,
worin * die Verknüpfung zur angrenzenden Carbonyl-Gruppe markiert und
- R³: Fluor, Chlor, Cyano, Methyl, Isopropyl, Methoxy oder Ethoxy bedeutet,
- R⁴: Wasserstoff, Fluor oder Chlor bedeutet,
- R⁵: Wasserstoff, Chlor oder Brom bedeutet
und
- R⁶: Methoxy, Difluormethoxy, Trifluormethoxy oder Isopropoxy bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Erfindungsgemäße stabile pharmazeutische Formulierung sind auch solche Formulierungen, in denen der mindestens eine Inhibitor des TASK-1 und/oder TASK-3 Kanals ausgewählt ist aus Verbindungen der Tabelle 1. Die Synthese dieser Verbindungen ist in der PCT/EP2016/079973 beschrieben.

**Tabelle 1: Verbindungen aus PCT/EP2016/079973**

| **Beispiel** | **Name** |
|---|---|
| **1** | (4-{[2-(4-Bromphenyl)imidazo[1,2-a]pyridin-3-yl]methyl}piperazin-1-yl)(cyclopentyl)methanon |
| **2** | (4-{[2-(4-Chlorphenyl)imidazo[1,2-a]pyridin-3-yl]methyl}piperazin-1-yl)(cyclopentyl)methanon |
| **3** | (4-{[2-(4-Chlorphenyl)imidazo[1,2-a]pyridin-3-yl]methyl}piperazin-1-yl)(6-methoxypyridin-2-yl)methanon |
| **4** | (4-{[2-(4-Bromphenyl)imidazo[1,2-a]pyridin-3-yl]methyl}piperazin-1-yl)(2-fluorphenyl)methanon |
| **5** | (4-{[2-(4-Bromphenyl)imidazo[1,2-a]pyridin-3-yl]methyl}piperazin-1-yl)(3-methoxyphenyl)methanon |
| **6** | (4-{[2-(4-Bromphenyl)imidazo[1,2-a]pyridin-3-yl]methyl}piperazin-1-yl)(2-chlor-5-fluorphenyl)methanon |
| **7** | (4-{[2-(4-Chlorphenyl)imidazo[1,2-a]pyridin-3-yl]methyl}piperazin-1-yl)(2-fluorphenyl)methanon |
| **8** | (4-{[2-(4-Fluorphenyl)imidazo[1,2-a]pyridin-3-yl]methyl}piperazin-1-yl)(cyclohexyl)methanon |
| **9** | (4-{[2-(4-Bromphenyl)imidazo[1,2-a]pyridin-3-yl]methyl}piperazin-1-yl)(cyclohexyl)methanon |
| **10** | (4-{[2-(4-Bromphenyl)imidazo[1,2-a]pyridin-3-yl]methyl}piperazin-1-yl)(tetrahydrofuran-3-yl)methanon |
| **11** | (4-{[2-(4-Bromphenyl)imidazo[1,2-a]pyridin-3-yl]methyl}piperazin-1-yl)(cyclobutyl)methanon |
| **12** | (4-{[2-(4-Bromphenyl)imidazo[1,2-a]pyridin-3-yl]methyl}piperazin-1-yl)(2-methoxyphenyl)methanon |
| **13** | (4-{[2-(4-Bromphenyl)imidazo[1,2-a]pyridin-3-yl]methyl}piperazin-1-yl)(5-fluor-2-methoxyphenyl)methanon |
| **14** | (4-{[2-(4-Bromphenyl)imidazo[1,2-a]pyridin-3-yl]methyl}piperazin-1-yl)(2-methylphenyl)methanon |
| **15** | (4-{[2-(4-Bromphenyl)imidazo[1,2-a]pyridin-3-yl]methyl}piperazin-1-yl)(5-fluor-2-methylphenyl)methanon |
| **16** | (2-Chlor-5-fluorphenyl)(4-{[2-(4-chlorphenyl)imidazo[1,2-a]pyridin-3-yl]methyl}piperazin-1-yl)methanon |
| **17** | (4-{[2-(4-Chlorphenyl)imidazo[1,2-a]pyridin-3-yl]methyl}piperazin-1-yl)(cyclohexyl)methanon |
| **18** | ((4-{[2-(4-Chlorphenyl)imidazo[1,2-a]pyridin-3-yl]methyl}piperazin-1-yl)(cyclobutyl)methanon |
| **19** | (4-{[2-(4-Chlorphenyl)imidazo[1,2-a]pyridin-3-yl]methyl}piperazin-1-yl)(3-methoxyphenyl)methanon |
| **20** | (4-{[2-(4-Chlorphenyl)imidazo[1,2-a]pyridin-3-yl]methyl}piperazin-1-yl)(2-methoxyphenyl)methanon |
| **21** | (4-{[2-(4-Chlorphenyl)imidazo[1,2-a]pyridin-3-yl]methyl}piperazin-1-yl)(5-fluor-2-methoxyphenyl)methanon |
| **22** | (4-{[2-(4-Chlorphenyl)imidazo[1,2-a]pyridin-3-yl]methyl}piperazin-1-yl)(2-methylphenyl)methanon |
| **23** | (4-{[2-(4-Chlorphenyl)imidazo[1,2-a]pyridin-3-yl]methyl}piperazin-1-yl)(5-fluor-2-methylphenyl)methanon |
| **24** | (4-{[2-(4-Chlorphenyl)imidazo[1,2-a]pyridin-3-yl]methyl}piperazin-1-yl)[3-(trifluormethoxy)phenyl]methanon |
| **25** | (4-{[2-(4-Chlorphenyl)imidazo[1,2-a]pyridin-3-yl]methyl}piperazin-1-yl)[3-(trifluormethyl)phenyl]methanon |
| **26** | ((4-{[2-(4-Chlorphenyl)imidazo[1,2-a]pyridin-3-yl]methyl}piperazin-1-yl)(pyridin-2-yl)methanon |
| **27** | (4-{[2-(4-Chlorphenyl)imidazo[1,2-a]pyridin-3-yl]methyl}piperazin-1-yl)(2-fluor-5-methoxyphenyl)methanon |
| **28** | (4-{[2-(4-Chlorphenyl)imidazo[1,2-a]pyridin-3-yl]methyl}piperazin-1-yl)(2-ethoxyphenyl)methanon |
| **29** | (2-Chlor-5-methoxyphenyl)(4-{[2-(4-chlorphenyl)imidazo[1,2-a]pyridin-3-yl]methyl}piperazin-1-yl)methanon |
| **30** | (4-{[2-(4-Chlorphenyl)imidazo[1,2-a]pyridin-3-yl]methyl}piperazin-1-yl)(tetrahydro-2*H*-pyran-2-yl)methanon |
| **31** | (4-{[2-(4-Chlorphenyl)imidazo[1,2-a]pyridin-3-yl]methyl}piperazin-1-yl)(3-isopropoxyphenyl)methanon |
| **32** | 2-[(4-{[2-(4-Chlorphenyl)imidazo[1,2-a]pyridin-3-yl]methyl}piperazin-1-yl)carbonyl]benzonitril |
| **33** | (4-{[2-(4-Chlorphenyl)imidazo[1,2-a]pyridin-3-yl]methyl}piperazin-1-yl)(3-isopropylphenyl)methanon |
| **34** | (4-{[2-(4-Chlorphenyl)imidazo[1,2-a]pyridin-3-yl]methyl}piperazin-1-yl)(2-isopropylphenyl)methanon |
| **35** | (4-{[2-(4-Chlorphenyl)imidazo[1,2-a]pyridin-3-yl]methyl}piperazin-1-yl)(tetrahydrofuran-2-yl)methanon |
| **36** | (3-Chlorphenyl)(4-{[2-(4-chlorphenyl)imidazo[1,2-a]pyridin-3-yl]methyl}piperazin-1-yl)methanon |
| **37** | (2-Chlorphenyl)(4-{[2-(4-chlorphenyl)imidazo[1,2-a]pyridin-3-yl]methyl}piperazin-1-yl)methanon |
| **38** | (4-{[2-(4-Chlorphenyl)imidazo[1,2-a]pyridin-3-yl]methyl}piperazin-1-yl)[6-(2,2,2-trifluorethoxy)pyridin-2-yl]methanon |
| **39** | (4-{[2-(4-Chlorphenyl)imidazo[1,2-a]pyridin-3-yl]methyl}piperazin-1-yl)(6-isopropoxypyridin-2-yl)methanon |
| **40** | (4-{[2-(4-Chlorphenyl)imidazo[1,2-a]pyridin-3-yl]methyl}piperazin-1-yl)(6-methoxy-4-methylpyridin-2-yl)methanon |
| **41** | (4-{[2-(4-Chlorphenyl)imidazo[1,2-a]pyridin-3-yl]methyl}piperazin-1-yl)[6-(cyclobutyloxy)pyridin-2-yl]methanon |
| **42** | (3-Brom-6-methoxypyridin-2-yl)(4-{[2-(4-chlorphenyl)imidazo[1,2-a]pyridin-3-yl]methyl}piperazin-1-yl)methanon |
| **43** | (3-Chlor-6-methoxypyridin-2-yl)(4-{[2-(4-chlorphenyl)imidazo[1,2-a]pyridin-3-yl]methyl}piperazin-1-yl)methanon |
| **44** | (4-{[2-(4-Chlorphenyl)imidazo[1,2-a]pyridin-3-yl]methyl}piperazin-1-yl)[6-(difluormethoxy)pyridin-2-yl]methanon |
| **45** | (4-{[2-(4-Chlorphenyl)imidazo[1,2-a]pyridin-3-yl]methyl}piperazin-1-yl)(6-ethoxypyridin-2-yl)methanon |
| **46** | (4-{[2-(4-Chlorphenyl)imidazo[1,2-a]pyridin-3-yl]methyl}piperazin-1-yl)[6-(tetrahydro-2*H*-pyran-4-yloxy)pyridin-2-yl]methanon |
| **47** | (4-{[2-(4-Bromphenyl)imidazo[1,2-a]pyridin-3-yl]methyl}piperazin-1-yl)(6-methoxypyridin-2-yl)methanon |
| **48** | (4-{[2-(4-Fluorphenyl)imidazo[1,2-a]pyridin-3-yl]methyl}piperazin-1-yl)(cyclopentyl)methanon |
| **49** | (4-{[2-(4-Fluorphenyl)imidazo[1,2-a]pyridin-3-yl]methyl}piperazin-1-yl)(cyclobutyl)methanon |
| **50** | (5-Fluor-2-methoxyphenyl)(4-{[2-(4-fluorphenyl)imidazo[1,2-a]pyridin-3-yl]methyl}piperazin-1-yl)methanon |
| **51** | (2-Chlor-5-fluorphenyl)(4-{[2-(4-fluorphenyl)imidazo[1,2-a]pyridin-3-yl]methyl}piperazin-1-yl)methanon |
| **52** | (4-{[2-(4-Fluorphenyl)imidazo[1,2-a]pyridin-3-yl]methyl}piperazin-1-yl)(2-methoxyphenyl)methanon |
| **53** | (2-Fluorphenyl)(4-{[2-(4-isopropylphenyl)imidazo[1,2-a]pyridin-3-yl]methyl}piperazin-1-yl)methanon |
| **54** | Cyclopentyl(4-{[2-(4-isopropylphenyl)imidazo[1,2-a]pyridin-3-yl]methyl}piperazin-1-yl)methanon |
| **55** | (4-{[2-(4-Isopropylphenyl)imidazo[1,2-a]pyridin-3-yl]methyl}piperazin-1-yl)(6-methoxypyridin-2-yl)methanon |
| **56** | Cyclopentyl(4-{[2-(4-methylphenyl)imidazo[1,2-a]pyridin-3-yl]methyl}piperazin-1-yl)methanon |
| **57** | Cyclohexyl(4-{[2-(4-methylphenyl)imidazo[1,2-a]pyridin-3-yl]methyl}piperazin-1-yl)methanon |
| **58** | (2-Methoxyphenyl)(4-{[2-(4-methylphenyl)imidazo[1,2-a]pyridin-3-yl]methyl}piperazin-1-yl)methanon |
| **59** | (6-Methoxypyridin-2-yl)(4-{[2-(4-methylphenyl)imidazo[1,2-a]pyridin-3-yl]methyl}piperazin-1-yl)methanon |
| **60** | (4-(3-{[4-(2-Fluorbenzoyl)piperazin-1-yl]methyl}imidazo[1,2-a]pyridin-2-yl)benzonitril |
| **61** | 4-[3-({4-[(6-Methoxypyridin-2-yl)carbonyl]piperazin-1-yl}methyl)imidazo[1,2-a]pyridin-2-yl]benzonitril |
| **62** | 4-(3-{[4-(Cyclopentylcarbonyl)piperazin-1-yl]methyl}imidazo[1,2-a]pyridin-2-yl)benzonitril |
| **63** | 4-(3-{[4-(Cyclohexylcarbonyl)piperazin-1-yl]methyl}imidazo[1,2-a]pyridin-2-yl)benzonitril |
| **64** | (4-{[2-(4-*tert.*-Butylphenyl)imidazo[1,2-a]pyridin-3-yl]methyl}piperazin-1-yl)(6-methoxypyridin-2-yl)methanon |
| **65** | (4-{[2-(4-*tert*.-Butylphenyl)imidazo[1,2-a]pyridin-3-yl]methyl}piperazin-1-yl)(2-fluorphenyl)methanon |
| **66** | (4-{[2-(4-*tert*.-Butylphenyl)imidazo[1,2-a]pyridin-3-yl]methyl}piperazin-1-yl)(cyclopentyl)methanon |
| **67** | (4-{[2-(4-Chlorphenyl)imidazo[1,2-a]pyridin-3-yl]methyl}piperazin-1-yl)[6-(trifluormethoxy)pyridin-2-yl]methanon |
| **68** | (4-{[2-(4-Chlorphenyl)imidazo[1,2-a]pyridin-3-yl]methyl}piperazin-1-yl)(3-fluor-6-methoxypyridin-2-yl)methanon |
| **69** | (4-{[2-(4-Cyclopropylphenyl)imidazo[1,2-a]pyridin-3-yl]methyl}piperazin-1-yl)(2-fluorphenyl)methanon |
| **70** | 4-(3-{[4-(2-Fluor-5-methoxybenzoyl)piperazin-1-yl]methyl}imidazo[1,2-a]pyridin-2-yl)benzonitril |
| **71** | 4-[3-({4-[(6-Methoxy-3-methylpyridin-2-yl)carbonyl]piperazin-1-yl}methyl)imidazo[1,2-a]pyridin-2-yl]benzonitril |
| **72** | (4-{[2-(4-Chlorphenyl)imidazo[1,2-a]pyridin-3-yl]methyl}piperazin-1-yl)(6-methoxy-3-methylpyridin-2-yl)methanon |
| **73** | (4-{[2-(4-*tert*.-Butylphenyl)imidazo[1,2-a]pyridin-3-yl]methyl}piperazin-1-yl)(6-methoxy-3-methylpyridin-2-yl)methanon |
| **74** | (4-{[2-(4-Bromphenyl)imidazo[1,2-a]pyridin-3-yl]methyl}piperazin-1-yl)(6-methoxy-3-methylpyridin-2-yl)methanon |

Erfindungsgemäße stabile pharmazeutische Formulierung sind auch solche Formulierungen, in denen der mindestens eine Inhibitor des TASK-1 und/oder TASK-3 Kanals ausgewählt ist aus der Gruppe bestehend aus

| **Beispiel** | **Name** |
|---|---|
| **1** | (4-{[2-(4-Bromphenyl)imidazo[1,2-a]pyridin-3-yl]methyl}piperazin-1-yl)(cyclopentyl)methanon |
| **2** | (4-{[2-(4-Chlorphenyl)imidazo[1,2-a]pyridin-3-yl]methyl}piperazin-1-yl)(cyclopentyl)methanon |
| **3** | (4-{[2-(4-Chlorphenyl)imidazo[1,2-a]pyridin-3-yl]methyl}piperazin-1-yl)(6-methoxypyridin-2-yl)methanon |
| **4** | (4-{[2-(4-Bromphenyl)imidazo[1,2-a]pyridin-3-yl]methyl}piperazin-1-yl)(2-fluorphenyl)methanon |

sowie ihre Salze, Solvate und Solvate der Salze.

Erfindungsgemäße stabile pharmazeutische Formulierung sind auch solche Formulierungen, in denen der mindestens eine Inhibitor des TASK-1 und/oder TASK-3 Kanals (4-{[2-(4-Chlorphenyl)imidazo[1,2-a]pyridin-3-yl]methyl}piperazin-1-yl)(6-methoxypyridin-2-yl)methanon ist.

Eine weitere Ausführungsform der vorliegenden Erfindung sind die erfindungsgemäßen stabilen pharmazeutischen Formulierungen zur nasalen oder pharyngealen Applikation zur Behandlung und/oder Prävention von Krankheiten.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen, wobei die nasale oder pharyngeale Applikation mit Hilfe von Nasensprays, Nasentropfen, Nasenlösungen, Pulverinhalatoren, Nebulizern, Dosieraerosolen oder halbfesten Gelen erfolgt.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen verwendet, wobei die Wirkdauer mindestens 3 Stunden beträgt.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen verwendet, wobei die Wirkdauer mindestens 4 Stunden beträgt.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen verwendet, wobei die Wirkdauer mindestens 5 Stunden beträgt.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von obstruktiven Schlafapnoen oder Schnarchen, enthaltend:
eine therapeutisch wirksame Menge des Inhibitors des TASK-1 und/oder TASK-3 Kanals 4-{[2-(4-Chlorphenyl)imidazo[1,2-a]pyridin-3-yl]methyl}piperazin-1-yl)(6-methoxypyridin-2-yl)-methanon oder ein Hydrat, Solvat, Polymorph oder Metabolit hiervon oder ein pharmazeutisch verwendbares Salz hiervon in 20% bis 100% w/v PEG400 und 0 bis 10% w/v Polysorbat 80 und 0 bis 80% w/v eines Phosphatpuffers, der einen pH-Wert von 7 aufweist, und gegebenenfalls mindestens einen weiteren Hilfsstoff, wobei die Wirkdauer der stabilen pharmazeutischen Formulierung nach nasaler oder pharyngealer Applikation mindestens 3 Stunden oder mindestens 4 Stunden oder mindestens 5 Stunden oder mindestens 6 Stunden oder mindestens 7 Stunden oder mindestens 8 Stunden beträgt.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von obstruktiven Schlafapnoen oder Schnarchen, enthaltend eine therapeutisch wirksame Menge des Inhibitors des TASK-1 und/oder TASK-3 Kanals 4-{[2-(4-Chlorphenyl)imidazo[1,2-a]pyridin-3-yl]methyl}piperazin-1-yl)(6-methoxy-pyridin-2-yl)-methanon oder ein Hydrat, Solvat, Polymorph oder Metabolit hiervon oder ein pharmazeutisch verwendbares Salz hiervon in 20% bis 100% w/v PEG400 und 0 bis 10% w/v Polysorbat 80 und 0 bis 80% w/v eines Phosphatpuffers, der einen pH-Wert von 7 aufweist, und gegebenenfalls mindestens einen weiteren Hilfsstoff, wobei die Wirkdauer der stabilen pharmazeutischen Formulierung nach nasaler oder pharyngealer Applikation mindestens 3 Stunden beträgt.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von obstruktiven Schlafapnoen oder Schnarchen, enthaltend:
eine therapeutisch wirksame Menge des Inhibitors des TASK-1 und/oder TASK-3 Kanals 4-{[2-(4-Chlorphenyl)imidazo[1,2-a]pyridin-3-yl]methyl}piperazin-1-yl)(6-methoxypyridin-2-yl)-methanon oder ein Hydrat, Solvat, Polymorph oder Metabolit hiervon oder ein pharmazeutisch verwendbares Salz hiervon in 20% bis 100% w/v PEG400 und 0 bis 10% w/v Polysorbat 80 und 0 bis 80% w/v eines Phosphatpuffers, der einen pH-Wert von 7 aufweist, und gegebenenfalls mindestens einen weiteren Hilfsstoff, wobei die Wirkdauer der stabilen pharmazeutischen Formulierung nach nasaler oder pharyngealer Applikation mindestens 4 Stunden beträgt.

Eine weitere Ausführungsform der vorliegenden Erfindung sind die erfindungsgemäßen stabilen pharmazeutischen Formulierungen zur nasalen oder pharyngealen Applikation zur Verwendung in einem Verfahren zur Behandlung und/oder Prävention von obstruktiven Schlafapnoen oder Schnarchen, enthaltend:
eine therapeutisch wirksame Menge des Inhibitors des TASK-1 und/oder TASK-3 Kanals 4-{[2-(4-Chlorphenyl)imidazo[1,2-a]pyridin-3-yl]methyl}piperazin-1-yl)(6-methoxypyridin-2-yl)-methanon oder ein Hydrat, Solvat, Polymorph oder Metabolit hiervon oder ein pharmazeutisch verwendbares Salz hiervon in 20% bis 100% w/v PEG400 und 0 bis 10% w/v Polysorbat 80 und 0 bis 80% w/v eines Phosphatpuffers, der einen pH-Wert von 7 aufweist, und gegebenenfalls mindestens einen weiteren Hilfsstoff, wobei die Wirkdauer der stabilen pharmazeutischen Formulierung nach nasaler oder pharyngealer Applikation mindestens 5 Stunden beträgt.

Die erfindungsgemäßen Formulierungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Formulierungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:
- Atemstimulantien, wie beispielhaft und vorzugsweise Theophyllin, Doxapram, Nicethamid oder Coffein;
- psychostimulierende Verbindungen, wie beispielhaft und vorzugsweise Modafinil oder Armodafinil;
- Amphetamine und Amphetamin-Derivate, wie beispielhaft und vorzugsweise Amphetamin, Metamphetamin oder Methylphenidat;
- Serotonin-Wiederaufnahmehemmer, wie beispielhaft und vorzugsweise Fluoxetin, Paroxetin, Citalopram, Escitalopram, Sertralin, Fluvoxamin oder Trazodon;
- Serotonin-Präkursoren, wie beispielhaft und vorzugsweise L-Tryptophan;
- selektive Serotonin-Noradrenalin-Wiederaufnahmehemmer, wie beispielhaft und vorzugsweise Venlafaxin oder Duloxetin;
- noradrenerge und spezifisch serotonerge Antidepressiva, wie beispielhaft und vorzugsweise Mirtazapin;
- selektive Noradrenalin-Wiederaufnahmehemmer, wie beispielhaft und vorzugsweise Reboxetin;
- tricyclische Antidepressiva, wie beispielhaft und vorzugsweise Amitriptylin, Protriptylin, Doxepin, Trimipramin, Imipramin, Clomipramin oder Desipramin;
- alpha2-adrenerge Agonisten, wie beispielhaft und vorzugsweise Clonidin;
- GABA-Agonisten, wie beispielhaft und vorzugsweise Baclofen;
- alpha-Sympathomimetika, wie beispielhaft und vorzugsweise Xylometazolin, Oxymetazolin, Phenylephrin, Naphazolin, Tetryzolin oder Tramazolin;
- Glucocorticoide, wie beispielhaft und vorzugsweise Fluticason, Budesonid, Beclometason, Mometason, Tixocortol oder Triamcinolon;
- Cannabinoid-Rezeptor-Agonisten;
- Carboanhydrase-Hemmer, wie beispielhaft und vorzugsweise Acetazolamid, Methazolamid oder Diclofenamid;
- Opioid- und Benzodiazepin-Rezeptor-Antagonisten, wie beispielhaft und vorzugsweise Flumazenil, Naloxon oder Naltrexon;
- Cholinesterase-Hemmer, wie beispielhaft und vorzugsweise Neostigmin, Pyridostigmin, Physostigmin, Donepezil, Galantamin oder Rivastigmin;
- *N*-Methyl-D-Aspartat- und Glutamat-Antagonisten, wie beispielhaft und vorzugsweise Amantadin, Memantin oder Sabeluzol;
- Nikotin-Rezeptor-Agonisten;
- Leukotrien-Rezeptor-Antagonisten, wie beispielhaft und vorzugsweise Montelukast oder Tripelukast;
- Dopamin-Rezeptor-Antagonisten, wie beispielhaft und vorzugsweise Dromperidon, Metoclopramid oder Benzamid-, Butyrophenon- oder Phenothiazin-Derivate;
- Appetitzügler, wie beispielhaft und vorzugsweise Sibutramin, Topiramat, Phentermin, Lipase-Inhibitoren oder Cannabinoid-Rezeptor-Antagonisten;
- Protonenpumpen-Inhibitoren, wie beispielhaft und vorzugsweise Pantoprazol, Omeprazol, Esomeprazol, Lansoprazol oder Rabeprazol;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil, Tadalafil, Udenafil, Dasantafil, Avanafil, Mirodenafil oder Lodenafil;
- NO- und Häm-unabhängige Aktivatoren der löslichen Guanylatcyclase (sGC), wie insbesondere die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der löslichen Guanylatcyclase (sGC), wie insbesondere Riociguat, Vericiguat sowie die in WO 00/06568, WO 00/06569, WO 02/ 42301, WO 03/095451, WO 2011/147809, WO 2012/004258, WO 2012/028647 und WO 2012/ 059549 beschriebenen Verbindungen;
- Prostacyclin-Analoga und IP-Rezeptor-Agonisten, wie beispielhaft und vorzugsweise Iloprost, Beraprost, Treprostinil, Epoprostenol oder Selexipag;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-HT_{2B}-Rezeptors wie PRX-08066;
- Antagonisten von Wachstumsfaktoren, Zytokinen und Chemokinen, beispielhaft und vorzugsweise Antagonisten von TGF-β, CTGF, IL-1, IL-4, IL-5, IL-6, IL-8, IL-13 und Integrinen;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- den Energiestoffwechsel des Herzens beeinflussende Verbindungen, wie beispielhaft und vorzugsweise Etomoxir, Dichloracetat, Ranolazin oder Trimetazidin;
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren, wie beispielhaft und vorzugsweise Nintedanib, Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib oder Tandutinib;
- anti-obstruktiv wirkende Mittel, wie sie z. B. zur Therapie der chronisch-obstruktiven Lungenerkrankung (COPD) oder eines Asthma bronchiale eingesetzt werden, beispielhaft und vorzugsweise aus der Gruppe der inhalativ oder systemisch angewendeten beta-adrenergen Rezeptor-Agonisten (beta-Mimetika) und der inhalativ angewendeten anti-muscarinergen Substanzen;
- entzündungshemmende, immunmodulierende, immunsuppressive und/oder zytotoxische Mittel, beispielhaft und vorzugsweise aus der Gruppe der systemisch oder inhalativ angewendeten Corticosteroide sowie Dimethylfumarat, Fingolimod, Glatirameracetat, β-Interferone, Natalizumab, Teriflunomid, Mitoxantron, Immunglobuline, Acetylcystein, Montelukast, Tripelukast, Azathioprin, Cyclophosphamid, Hydroxycarbamid, Azithromycin, Interferon-γ, Pirfenidon oder Etanercept;
- antifibrotisch wirkende Mittel, wie beispielhaft und vorzugsweise Lysophosphatidsäure-Rezeptor 1 (LPA-1)-Antagonisten, CTGF-Inhibitoren, IL-4-Antagonisten, IL-13-Antagonisten, TGF-β-Antagonisten oder Pirfenidon;
- antithrombotisch wirkende Mittel, beispielhaft und vorzugsweise aus der Gruppe der Thrombozytenaggregationshemmer, der Antikoagulantien und der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Formulierungen in Kombination mit einem beta-adrenergen Rezeptor-Agonisten, wie beispielhaft und vorzugsweise Albuterol, Isoproterenol, Metaproterenol, Terbutalin, Fenoterol, Formoterol, Reproterol, Salbutamol oder Salmeterol, verabreicht.

Bei einer bevorzugten Ausführungsform der Erfindung werden die erfindungsgemäßen Formulierungen in Kombination mit einer anti-muscarinergen Substanz, wie beispielhaft und vorzugsweise Ipratropiumbromid, Tiotropiumbromid oder Oxitropiumbromid, verabreicht.

Bei einer bevorzugten Ausführungsform der Erfindung werden die erfindungsgemäßen Formulierungen in Kombination mit einem Corticosteroid, wie beispielhaft und vorzugsweise Prednison, Prednisolon, Methylprednisolon, Triamcinolon, Dexamethason, Betamethason, Beclometason, Flunisolid, Budesonid oder Fluticason, verabreicht.

Unter antithrombotisch wirkenden Mittel werden vorzugsweise Verbindungen aus der Gruppe der Thrombozytenaggregationshemmer, der Antikoagulantien und der profibrinolytischen Substanzen verstanden.

Bei einer bevorzugten Ausführungsform der Erfindung werden die erfindungsgemäßen Formulierungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Formulierungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Formulierungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Formulierungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Formulierungen in Kombination mit Heparin oder einem low molecular weight (LMW)-Heparin-Derivat verabreicht.

Bei einer bevorzugten Ausführungsform der Erfindung werden die erfindungsgemäßen Formulierungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Formulierungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Formulierungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Formulierungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Formulierungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Formulierungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Formulierungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Formulierungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Formulierungen in Kombination mit einem Mineralocorticoid-Rezeptor-Antagonisten, wie beispielhaft und vorzugsweise Spironolacton, Eplerenon oder Finerenon, verabreicht.

Bei einer bevorzugten Ausführungsform der Erfindung werden die erfindungsgemäßen Formulierungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerolin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Formulierungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Formulierungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Formulierungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Formulierungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Formulierungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Formulierungen in Kombination mit einem MTP-Inhibitor, wie beispielhaft und vorzugsweise Implitapid, BMS-201038, R-103757 oder JTT-130, verabreicht.

Bei einer bevorzugten Ausführungsform der Erfindung werden die erfindungsgemäßen Formulierungen in Kombination mit einem PPAR-gamma-Agonisten, wie beispielhaft und vorzugsweise Pioglitazon oder Rosiglitazon, verabreicht.

Bei einer bevorzugten Ausführungsform der Erfindung werden die erfindungsgemäßen Formulierungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Formulierungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Formulierungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Formulierungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Formulierungen in Kombination mit einem Gallensäure-Reabsorptionshemmer, wie beispielhaft und vorzugsweise ASBT (= IBAT)-Inhibitoren wie z. B. AZD-7806, S-8921, AK-105, BARI-1741, SC-435 oder SC-635, verabreicht.

Bei einer bevorzugten Ausführungsform der Erfindung werden die erfindungsgemäßen Formulierungen in Kombination mit einem Lipoprotein(a)-Antagonisten, wie beispielhaft und vorzugsweise Gemcabene calcium (CI-1027) oder Nicotinsäure, verabreicht.

Besonders bevorzugt sind Kombinationen der erfindungsgemäßen Formulierungen mit einem oder mehreren weiteren Wirkstoffen ausgewählt aus der Gruppe bestehend aus Atemstimulantien, psychostimulierenden Verbindungen, Serotonin-Wiederaufnahmehemmern, noradrenergen, serotonergen und tricyclischen Antidepressiva, sGC-Stimulatoren, Mineralocorticoid-Rezeptor-Antagonisten, entzündungshemmend wirkenden Mitteln, immunmodulierend wirkenden Mitteln, immunsuppressiv wirkenden Mitteln und zytotoxisch wirkenden Mitteln.

Die erfindungsgemäßen Formulierungen können bei Bedarf auch in Zusammenhang mit dem Einsatz eines oder mehrerer medizinisch-technischer Geräte oder Hilfsmittel verwendet werden, solange dies nicht zu unerwünschten und inakzeptablen Nebeneffekten führt. Für eine solche Kombinationsanwendung in Betracht kommende medizinische Geräte und Hilfsmittel sind beispielhaft und vorzugsweise:
- Geräte zur Atemwegs-Überdruckbeatmung, wie beispielhaft und vorzugsweise CPAP (*continuous positive airway pressure*)*-Geräte,* BiPAP (*bilevel positive airway pressure*)*-*Geräte und IPPV (*intermittent positive pressure ventilation*)-*Geräte;*
- Neurostimulatoren des *Nervus hypoglossus;*
- intraorale Hilfsmittel, wie beispielhaft und vorzugsweise Protrusionsspangen;
- nasale Einwegventile;
- Nasenstents.

Gemäß einer Ausführungsform beträgt die Dosierung bei intranasaler Applikation etwa 0.1 µg bis 500 µg pro Tag. Gemäß einer weiteren Ausführungsform beträgt die Dosierung bei intranasaler Applikation etwa 1 µg bis 250 µg pro Tag. Gemäß einer weiteren Ausführungsform beträgt die Dosierung bei intranasaler Applikation etwa 1 µg bis 120 µg pro Tag. Gemäß einer weiteren Ausführungsform wird die Dosis von etwa 0.1 µg bis 500 µg pro Tag oder von etwa 1 µg bis 250 µg pro Tag oder von etwa 1 µg bis 120 µg pro Tag, einmal täglich vor dem Schlafen intranasal appliziert. Gemäß einer Ausführungsform wird die Dosis von etwa 0.1 µg bis 500 µg pro Tag oder von etwa 1 µg bis 250 µg pro Tag oder von etwa 1 µg bis 120 µg pro Tag, einmal täglich je zur Hälfte in jede Nasenöffnung appliziert. Gemäß einer Ausführungsform wird die Dosis von etwa 0.1 µg bis 500 µg pro Tag oder von etwa 1 µg bis 250 µg pro Tag oder von etwa 1 µg bis 120 µg pro Tag, einmal täglich vor dem Schlafen je zur Hälfte in jede Nasenöffnung appliziert.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Bewertung der pharmakologischen Wirksamkeit

**Abkürzungsverzeichnis**

| | |
|---|---|
| AHI | Apnoe-Hypopnoe Index |
| Na-CMC | Na-Carboxymethylcellulose |
| CMC | kritische Mizellenbildungskonzentration (critical micelle concentration) |
| CPAP System | Continuous Positive Airway Pressure System |
| EDTA | Ethylendiamintetraessigsäure (Ethylene Diamine Tetraacetic Acid) |
| EMG | Elektromyogramm |
| mPa*s | Millipascalsekunde |
| OSA | Obstruktive Schlafapnoe |
| PEG | Polyethylenglykol |
| TASK | TWIK-related acid-sensitive K⁺ channel |

Die pharmakologische Aktivität der in den erfindungsgemäßen Formulierungen enthaltenen Inhibitoren des TASK-1 und/oder TASK-3 Kanals wurde in der PCT/EP2016/079973 durch *in vitro* Versuche gezeigt.

Die pharmakologische Aktivität der erfindungsgemäßen Formulierungen kann durch *in vivo-*Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

### Tiermodell der obstruktiven Schlafapnoe am Schwein

Die Wirkungen der erfindungsgemäßen Formulierungen der Inhibitoren von TASK-1 und/oder TASK-3 Kanälen auf die Aktivierungsschwelle des Genioglossusmuskels durch negativen Druck und die Kollapsibilität der oberen Atemwege wurden an einem Schweinemodell für obstruktive Schlafapnoe untersucht.

Durch den Einsatz von Unterdruck kann bei anästhesierten, spontan-atmenden Schweinen ein Kollaps und damit ein Verschluss der oberen Atemwege ausgelöst werden [Wirth et al., Sleep 36, 699-708 (2013)].

Für das Modell wurden Deutsche Landschweine verwendet. Da sich beim Menschen in liegender, schlafender Position die nasale Achse in einer fast vertikalen Position befindet, wurden die Schweine bei den Versuchen in einer sitzenden Position (70 Grad) fixiert, wobei die Nase nach oben zeigte. Dadurch floss die Formulierung nach nasaler Gabe nach unten über alle Bereiche der oberen Atemwege. Die Schweine wurden anästhesiert und tracheotomiert. In den rostralen und kaudalen Teil der Trachea wurde jeweils eine Kanüle eingebunden. Die rostrale Kanüle wurde mittels eines T-Stückes einerseits mit einem Gerät verbunden, welches Unterdruck generiert, und andererseits mit der kaudalen Kanüle. Die kaudale Kanüle war mittels eines T-Stückes mit der rostralen Kanüle verbunden sowie mit einem Schlauch, der unter Umgehung der oberen Atemwege eine spontane Atmung erlaubte. Durch entsprechendes Verschließen und Öffnen der Schläuche war es so möglich, dass das Schwein von einer normalen Nasenatmung zu einer Atmung über die kaudale Kanüle wechseln konnte, während die oberen Atemwege isoliert und mit dem Gerät zur Erzeugung des Unterdrucks verbunden waren. Die Muskelaktivität des *Musculus genioglossus* wurde mittels Elektromyogramm (EMG) registriert.

Zu bestimmten Zeitpunkten wurde die Kollapsibilität der oberen Atemwege getestet, indem das Schwein über die kaudale Kanüle atmete und an die oberen Atemwege Unterdrücke von -50, -100 und -150 mbar (entsprechend -50, -100 und -150 cm Wassersäule (cm H₂O)) angelegt wurden. Hierdurch kollabierten die oberen Atemwege, was durch Unterbrechung des Luftstroms und einen Druckabfall im Schlauchsystem angezeigt wurde. Dieser Test wurde durchgeführt vor Gabe der Testsubstanz und in bestimmten Abständen nach Gabe die Testsubstanz. Eine entsprechend wirksame Testsubstanz konnte diesen Kollaps der Atemwege in der inspiratorischen Phase verhindern.

Nach dem Wechsel von nasaler Atmung zur Atmung durch die kaudale Kanüle war keine EMG-Aktivität des *Musculus genioglossus* beim anästhesierten Schwein messbar. Als ein weiterer Test wurde dann der Unterdruck ermittelt, bei dem die EMG-Aktivität wieder einsetzte. Dieser Schwellenwert wurde bei Wirksamkeit einer Testsubstanz zu positiveren Werten verschoben. Die Untersuchung wurde ebenfalls vor Gabe der Testsubstanz und zu bestimmten Zeitpunkten nach Gabe der Testsubstanz durchgeführt. Die Gabe der Testsubstanz erfolgte nasal.

Alle in den folgenden Tabellen gezeigten Ergebnisse wurden mit der in Tabelle 1 als Beispiel 3 aufgeführten Verbindung durchgeführt. Soweit nicht anders angegeben, wurden die Daten bei einem Unterdruck von -100 mbar (entsprechend -100 cm Wassersäule (cm H₂O)) auf die oberen Atemwege gemessen.

Der in Tabelle 1 als Beispiel 3 aufgeführte Wirkstoff wurde in den in der folgenden Tabelle 2 aufgeführten verschiedenen Formulierungen gelöst und in einer Menge von 0 µg, 3 µg bzw. 30 µg pro Schwein verabreicht. Die Wirkstoffformulierung bzw. das reine Vehikel wurde mit einer Pipette in einem Volumen von je 400 µl in jede Nasenöffnung appliziert.

**Tabelle 2: Zusammensetzungen der Formulierungen für die nasale Gabe, in der die in Tabelle 1 als Beispiel 3 aufgeführte Verbindung verabreicht wurde:**

| **Formulierung** | **Phosphatpuffer pH 7 [% w/v]** | **Polysorbat 80 [% w/v]** | **Glycerol 85% (Glycerol absolut) [% w/v]** | **PEG400 [% w/v]** | **Propylenglycol [% w/v]** | **Na-CMC [% w/v]** |
|---|---|---|---|---|---|---|
| 1 | 90 | 10 | | | | |
| 2 | | | | 100 | | |
| 3 | 70 | 10 | | 20 | | |
| 4 | 85 | 10 | | 5 | | |
| 5 | 87.5 | 10 | | 2.5 | | |
| 6 | 67.5 | 10 | 2.5 (2.125) | 20 | | |
| 7 | 70 | 10 | | | 20 | |
| 8 | 68.75 | 10 | | 20 | | 1.25 |
| 9 | 88.75 | 10 | | | | 1.25 |

Optional enthalten die Formulierungen von Tabelle 1 zusätzlich Butylhydroxyanisol in einer Konzentration von 0.02% w/v.

Die Herstellung des Phosphatpuffers pH 7, 0.063 M erfolgte nach European Pharmacopoeia 8.7: 5.18 g wasserfreies Dinatrium-Hydrogenphosphat und 3.65 g Natrium-Dihydrogenphosphat Monohydrat wurden in 950 mL Wasser gelöst, der pH-Wert wurde mit Phosphorsäure eingestellt und es wurde auf 1000 mL mit Wasser aufgefüllt. Alternativ wurde für die Herstellung des Phosphatpuffers Dinatrium-Hydrogenphosphats Dihydrat und Natrium-Dihydrogenphosphat Dihydrat anstelle des wasserfreien Dinatrium-Hydrogenphosphats und des Natrium-Dihydrogenphosphat Monohydrats verwendet. Hierfür wurden 6.49 g Dinatrium-Hydrogenphosphats Dihydrat und 4.13 g Natrium-Dihydrogenphosphat Dihydrat in 950 mL Wasser gelöst, der pH-Wert wurde mit Phosphorsäure eingestellt und es wurde auf 1000 mL mit Wasser aufgefüllt.

Die Wirkdauer ist in diesem Schweinemodell definiert als die Zeit [min], in der bei keinem Tier ein Kollaps der oberen Atemwege beobachtet wurde, als Mittelwert der angegebenen Anzahl von Tieren. Eine Wirkdauer, die als ">" X min angegeben ist bedeutet, dass der Versuch bei X min beendet wurde und bis dahin noch bei keinem Tier ein Kollaps der oberen Atemwege beobachtet wurde.

**Tabelle 3: Wirkdauer von Beispiel 3/Tabelle 1 in Phosphatpuffer pH7/Polysorbat 80 mit PEG400 (Formulierung 3) oder mit Glycerol 85% und PEG400 (Formulierung 6) im Vergleich zu der Wirkdauer von Beispiel 3/Tabelle 1 in Phosphatpuffer pH7/Polysorbat 80 (Formulierung 1)**

| **Formulierung nach Tabelle 1** | **PEG400 [% w/v]** | **Glycerol 85% (Glycerol absolut) [% w/v]** | **Beispiel 3 aus Tab. 1 [µg]** | **Wirkdauer [min], Mittelwert** |
|---|---|---|---|---|
| 1 | 0 | 0 | 3 | 150 |
| 1 | 0 | 0 | 30 | 120 |
| 3 | 20 | 0 | 0 | 0 |
| 3 | 20 | 0 | 3 | 180 |
| 3 | 20 | 0 | 30 | 300 |
| 6 | 20 | 2.5 (2.125) | 3 | 240 |

**Tabelle 4: Wirkdauer von Beispiel 3/Tabelle 1 in Phosphatpuffer pH7/Polysorbat 80/PEG, Vergleich verschiedener PEG Konzentrationen**

| **Formulierung nach Tabelle 1** | **PEG400 [% w/v]** | **Beispiel 3 aus Tabelle 1 [µg]** | **Wirkdauer [min] Mittelwert** |
|---|---|---|---|
| 2 | 100 | 3 | > 240 |
| 3 | 20 | 3 | 180 |
| 4 | 5 | 3 | 180 |
| 5 | 2.5 | 3 | 150 |
| 1 | 0 | 3 | 150 |

**Tabelle 5: Wirkdauer von Beispiel 3/Tabelle 1 in Phosphatpuffer pH7/Polysorbat 80 (90/10) + Na-CMC, Vergleich mit der Wirkdauer von Beispiel 3/Tabelle 1 in Phosphatpuffer pH7/Polysorbat 80/PEG400 (70/10/20)**

| **Formulierung nach Tabelle 1** | **Na-CMC/PEG400 [% w/v]** | **Beispiel 3 aus Tabelle 1 [µg]** | **Wirkdauer [min] Mittelwert** |
|---|---|---|---|
| 9 | 1.25 Na-CMC | 3 | 120 |
| 3 | 20 PEG | 3 | 180 |

**Tabelle 6: Wirkdauer von Beispiel 3/Tabelle 1 in Phosphatpuffer pH7/Polysorbat 80 mit PEG (70/10/20) oder Propylenglycol (70/10/20)**

| **Formulierung nach Tabelle 1** | **PEG400 / Propylenglykol [% w/v]** | **Beispiel 3 [µg]** | **Wirkdauer [min] Mittelwert** |
|---|---|---|---|
| 3 | 20 PEG | 30 | 300 |
| 7 | 20 Propylenglycol | 30 | 180 |

## Patentansprüche

1. Stabile pharmazeutische Formulierung zur nasalen oder pharyngealen Applikation enthaltend:
eine therapeutisch wirksame Menge mindestens eines Inhibitors des TASK-1 und/oder TASK-3 Kanals oder eines Hydrats, Solvats, Polymorphs oder Metaboliten hiervon oder eines pharmazeutisch verwendbaren Salzes hiervon in
>2.5% bis 100% w/v Polyethylenglykol und
gegebenenfalls mindestens einen Hilfsstoff,
wobei die Formulierung einen pH-Wert von 4 bis 8 aufweist.

2. Stabile pharmazeutische Formulierung zur nasalen oder pharyngealen Applikation nach Anspruch 1, wobei der gegebenenfalls mindestens eine Hilfsstoff ausgewählt ist aus der Gruppe bestehend aus mindestens einem pH-Regulator, mindestens einem Lösungsvermittler, mindestens einem Antioxidans, mindestens einem Stabilisator, mindestens einem Verdickungsmittel, mindestens einem Konservierungsmittel, mindestens einer Tonizitätseinstellenden Substanz, mindestens einem Aroma, mindestens einem Duftstoff und mindestens einem Farbstoff.

3. Stabile pharmazeutische Formulierung zur nasalen oder pharyngealen Applikation nach Anspruch 1 oder 2, wobei der gegebenenfalls mindestens eine pH-Regulator ausgewählt ist aus der Gruppe bestehend aus Citronensäure und ihren Salzen, Essigsäure und ihren Salzen, Phosphorsäure und ihren Salzen, Salzsäure, Carbonsäuren, Bicarbonsäuren, Aminosäuren, Oxocarbonsäuren, Polycarbonsäuren, Natriumhydroxid, Kaliumhydroxid, Natriumcarbonat und Natriumhydrogencarbonat.

4. Stabile pharmazeutische Formulierung zur nasalen oder pharyngealen Applikation nach einem der Ansprüche 1 bis 3, wobei der gegebenenfalls mindestens eine Lösungsvermittler ausgewählt ist aus der Gruppe bestehend aus Ethanol, Polysorbat 20, Polyoxyethylen(8)-stearat und Polysorbat 80.

5. Stabile pharmazeutische Formulierung zur nasalen oder pharyngealen Applikation nach einem der Ansprüche 1 bis 4, wobei das gegebenenfalls mindestens eine Antioxidans ausgewählt ist aus der Gruppe bestehend aus Citronensäure, Butylhydroxyanisol, Butylhydroxytoluol, EDTA und einer Begasung mit Stickstoff.

6. Stabile pharmazeutische Formulierung zur nasalen oder pharyngealen Applikation nach einem der Ansprüche 1 bis 5, wobei das gegebenenfalls mindestens eine Konservierungsmittel ausgewählt ist aus der Gruppe bestehend aus C₈-C₁₈ Alkoniumchlorid, Methylparaben, Propylparaben, Sorbinsäure, Chlorbutanol und Benzalkoniumchlorid.

7. Stabile pharmazeutische Formulierung zur nasalen oder pharyngealen Applikation nach einem der Ansprüche 1 bis 6, wobei die Formulierung 5 bis 100% w/v Polyethylenglykol 400, 0 bis 10% w/v eines Lösungsvermittlers, 0 bis 95% w/v eines pH-Regulators und gegebenenfalls mindestens einen weiteren Hilfsstoff enthält.

8. Stabile pharmazeutische Formulierung zur nasalen oder pharyngealen Applikation nach einem der Ansprüche 1 bis 7, wobei der mindestens eine Inhibitor des TASK-1 und/oder TASK-3 Kanals ausgewählt ist aus einer Verbindung der Formel (I), 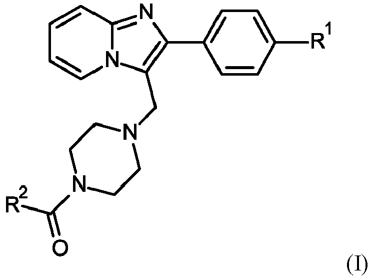 in welcher
R¹ für Halogen, Cyano, (C₁-C₄)-Alkyl, Cyclopropyl oder Cyclobutyl steht
und
R² für (C₄-C₆)-Cycloalkyl steht, worin eine Ring-CH₂-Gruppe gegen -O- ausgetauscht sein kann,
oder
für eine Phenyl-Gruppe der Formel (a) oder eine Pyridyl-Gruppe der Formel (b) 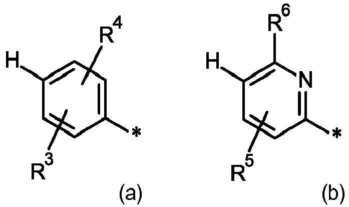 steht,
worin * die Verknüpfung zur angrenzenden Carbonyl-Gruppe markiert und
R³ Fluor, Chlor, Brom, Cyano, (C₁-C₃)-Alkyl oder (C₁-C₃)-Alkoxy bedeutet, wobei (C₁-C₃)-Alkyl und (C₁-C₃)-Alkoxy bis zu dreifach mit Fluor substituiert sein können,
R⁴ Wasserstoff, Fluor, Chlor, Brom oder Methyl bedeutet,
R⁵ Wasserstoff, Fluor, Chlor, Brom oder Methyl bedeutet
und
R⁶ Wasserstoff, (C₁-C₃)-Alkoxy, Cyclobutyloxy, Oxetan-3-yloxy, Tetrahydrofuran-3-yloxy oder Tetrahydro-2*H*-pyran-4-yloxy bedeutet, wobei (C₁-C₃)-Alkoxy bis zu dreifach mit Fluor substituiert sein kann,
sowie einem Hydrat, Solvat, Polymorph oder Metabolit hiervon oder einem pharmazeutisch verwendbaren Salz hiervon.

9. Stabile pharmazeutische Formulierung zur nasalen oder pharyngealen Applikation nach einem der Ansprüche 1 bis 8, wobei der mindestens eine Inhibitor des TASK-1 und/oder TASK-3 Kanals ausgewählt ist aus
(4-{[2-(4-Chlorphenyl)imidazo[1,2-a]pyridin-3-yl]methyl}piperazin-1-yl)(6-methoxypyridin-2-yl)¬methanon,
(4-{[2-(4-Bromphenyl)imidazo[1,2-a]pyridin-3-yl]methyl}piperazin-1-yl)(2-fluorphenyl)-methanon,
(4-{[2-(4-Bromphenyl)imidazo[1,2-a]pyridin-3-yl]methyl}piperazin-1-yl)(cyclopentyl)-methanon, und
(4-{[2-(4-Chlorophenyl)imidazo[1,2-a]pyridin-3-yl]methyl}piperazin-1-yl)(cyclopentyl)-methanon,
sowie einem Hydrat, Solvat, Polymorph oder Metabolit hiervon oder einem pharmazeutisch verwendbaren Salz hiervon.

10. Stabile pharmazeutische Formulierung nach einem der Ansprüche 1 bis 9, wobei der mindestens eine Inhibitor des TASK-1 und/oder TASK-3 Kanals (4-{[2-(4-Chlorphenyl)imidazo[1,2-a]pyridin-3-yl]methyl}piperazin-1-yl)(6-methoxy-pyridin-2-yl)methanon ist oder ein Hydrat, Solvat, Polymorph oder Metabolit hiervon oder ein pharmazeutisch verwendbares Salz hiervon.

11. Stabile pharmazeutische Formulierung zur nasalen oder pharyngealen Applikation nach einem der Ansprüche 1 bis 9 zur Behandlung und/oder Prävention von Krankheiten.

12. Stabile pharmazeutische Formulierung zur nasalen oder pharyngealen Applikation nach einem der Ansprüche 1 bis 9 zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen.

13. Stabile pharmazeutische Formulierung zur nasalen oder pharyngealen Applikation nach einem der Ansprüche 1 bis 9 zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen, wobei die nasale oder pharyngeale Applikation mit Hilfe von Nasensprays, Nasentropfen, Nasenlösungen, Pulverinhalatoren, Nebulizern, Dosieraerosolen oder halbfesten Gelen erfolgt.

14. Stabile pharmazeutische Formulierung zur nasalen oder pharyngealen Applikation nach einem der Ansprüche 1 bis 9 zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen, wobei die Wirkdauer mindestens 4 Stunden beträgt.

15. Stabile pharmazeutische Formulierung zur nasalen oder pharyngealen Applikation nach einem der Ansprüche 1 bis 9 zur Verwendung in einem Verfahren zur Behandlung und/oder Prävention von obstruktiven Schlafapnoen oder Schnarchen, enthaltend:
eine therapeutisch wirksame Menge des Inhibitors des TASK-1 und/oder TASK-3 Kanals 4-{[2-(4-Chlorphenyl)imidazo[1,2-a]pyridin-3-yl]methyl}piperazin-1-yl)(6-methoxypyridin-2-yl)-methanon oder ein Hydrat, Solvat, Polymorph oder Metabolit hiervon oder ein pharmazeutisch verwendbares Salz hiervon in
20% bis 100% w/v PEG400 und
0 bis 10% w/v Polysorbat 80 und 0 bis 80% w/v eines Phosphatpuffers, der einen pH-Wert von 7 aufweist, und gegebenenfalls mindestens einen weiteren Hilfsstoff,
wobei die Wirkdauer der stabilen pharmazeutischen Formulierung nach nasaler oder pharyngealer Applikation mindestens 5 Stunden beträgt.
